Europäisches Patentamt

European Patent Office

Office européen des brevets

(19)

(11) Publication number: **0 347 262**
**A1**

# EUROPEAN PATENT APPLICATION

(12)

(21) Application number: **89306176.2**

(22) Date of filing: **19.06.89**

(51) Int. Cl.⁴: **C 12 N 15/00**
**C 12 N 1/20, C 07 H 21/04,**
**C 07 K 13/00**
**//(C12N1/20,C12R1:19)**

(30) Priority: **17.06.88 US 208895**

(43) Date of publication of application:
**20.12.89 Bulletin 89/51**

(84) Designated Contracting States:
**AT BE CH DE ES FR GB GR IT LI LU NL SE**

(71) Applicant: **MOUNT SINAI SCHOOL OF MEDICINE OF THE CITY UNIVERSITY OF NEW YORK**
**One Gustave L. Levy Place**
**New York, NY 10029 (US)**

**YALE UNIVERSITY**
**451 College Street**
**New Haven Connecticut 06510 (US)**

(72) Inventor: **Nemerson, Yale**
**Box 550**
**Great Barrington Massachusetts 01230 (US)**

**Konigsberg, William**
**189 Everit Street**
**New Haven Connecticut 06511 (US)**

(74) Representative: **Lucas, Brian Ronald et al**
**Lucas, George & Co. 135 Westhall Road**
**Warlingham Surrey CR3 9HJ (GB)**

(54) Cloning and expression of human tissue factor.

(57) Recombinant cloning vectors replicable in a suitable host which contain a DNA fragment the sequence of which codes for human tissue factor, a procoagulant protein which triggers the extrinsic pathway of coagulation, are provided. Recombinant bacteriophage λ10,3 which contains a 2147 bp cDNA insert which codes for tissue factor was obtained by screening a human placental cDNA library cloned into λt11 for tissue factor coding sequences and purifying the recombinant bacteriophage containing said sequences. The 2147 bp cDNA fragment coding for human tissue factor and the substantially pure human tissue factor encoded therefrom are also provided. Also provided are a recombinant plasmid pKS-2B which contains the 2147 bp cDNA coding for tissue factor, as well as recombinant vectors which in suitable host cells express human tissue factor soluble human tissue factor and a truncated human tissue factor. Furthermore, substantially pure human tissue factor, novel soluble tissue factor and truncated human tissue factor are provided.

EP 0 347 262 A1

**Description**

## CLONING AND EXPRESSION OF HUMAN TISSUE FACTOR

### BACKGROUND OF THE INVENTION

The present invention relates to novel recombinant vectors replicable in a suitable host which contain cloned DNA sequences coding for human tissue factor. The invention also relates to the DNA coding for human tissue factor and to the substantially pure human tissue factor and functional portions thereof encoded therefrom. The invention also provides for transformant hosts containing expression vectors capable of expressing human tissue factor and a novel soluble form of human tissue factor and a novel truncated form of human tissue factor.

The coagulation (clotting) system in man and animals is a major contributor to the maintenance of hemostasis and also to thrombus (blood clot) formation. Coagulation is essentially a cascade in which each clotting factor, which is normally present in the blood and other tissues as an inactive enzyme precursor, i.e., zymogen, is in sequence activated into a proteolytic enzyme that selectively attacks the next zymogen in the clotting sequence, thereby converting it into an active enzyme. Amplification occurs at each step so that a small initial stimulus ultimately results in a significant amount of fibrin clot; which is the final product of the clotting process.

The clotting cascade begins as two separate pathways that ultimately converge. One pathway is "intrinsic" to the blood and the other one is termed "extrinsic" because it is triggered by clotting factors not normally present in blood. It is believed that the intrinsic pathway plays a major role in hemostasis following injury. The extrinsic pathway can become activated in a variety of pathologic situations, e.g., diffuse endothelial damage, advanced cancer, endotoxemia, and pregnancy complications.

There is now considerable evidence that coagulation is started in the body when factor VII, a vitamin K-dependent plasma clotting factor protein and tissue factor, a cell-bound protein not normally associated with blood cells, interact. (See e.g. Nemerson, Blood 71:1-8, 1988 for a review). This interaction results in an activated complex which has enzymatic activity and initiates clotting by converting two other proteins, i.e., factor X and factor IX, to their active, enzymatic forms, factor $X_a$ and factor $IX_a$, respectively. (In accord with common practice, the zymogen precursor forms of the active blood clotting factors are denoted by a Roman numeral, and the active forms are indicated by a subscripted "a", e.g. factor X for zymogen and factor $X_a$ for activated factor.)

Tissue factor is a procoagulant protein present on the surface of virtually all cells not normally in direct contact with blood. However, tissue factor is inducible in cultured endothelial cells and monocytes upon stimulation with various pharmacologic mediators, e.g. tumor necrosis factor, interleukin-1, and endotoxin. The extrinsic coagulation pathway is triggered by tissue factor which complexes with and activates factor VII, a vitamin-K dependent serine protease zymogen. The activation of factor VII by tissue factor, which is a blood clotting enzyme cofactor, occurs in the presence of calcium and results from a conformational change in factor VII. See, e.g., Nemerson et al., in Progress in Hemostasis and Thrombosis, Spaet, T.H. edit., Grune & Stratton, New York, vol. 6, pp. 237-261, 1982; Carson, Prog. Clin. Pathol. 9:1-14, 1984. The precise conformational change in factor VII which causes it to become activated and initiate clotting, however, has not yet been determined.

Tissue factor and factor VII are extremely important components of the clotting cascade. The severe bleeding frequently seen in individuals who are markedly deficient in factor VII demonstrates the physiologic significance of the extrinsic pathway of blood clotting. In contrast, individuals deficient in proteins involved in the early steps of the intrinsic pathway of coagulation, i.e., high molecular weight kininogen, prekallikrein, and factor XII, are asymptomatic.

Tissue factor, which is a cell membrane-bound glycoprotein associated with phospholipids, is not normally present in the circulation. When blood vessels are disrupted, however, factor VII, which is a plasma coagulation factor, can complex with tissue factor, thereby forming a catalytically-active species which activates both factor IX (plasma thromboplastin component), a component of the intrinsic pathway, to form factor $IX_a$ and factor X (Stuart factor), which is involved in both the extrinsic and intrinsic pathways of coagulation, to yield factor $X_a$. Tissue factor is thus an important component of the human clotting pathways. Human tissue factor has important use as a diagnostic reagent to monitor and study clotting disorders and potential use in anticoagulation. Chemical and biological characterization of human tissue factor is thus clearly important to an understanding of coagulation in humans.

Crude preparations of tissue factor are used in routine clinical laboratories to perform a test known as the Quick One-Stage Prothrombin Time. In this test, crude tissue factor, usually a mixture of acetone-dehydrated rabbit brain and lung, is added along with $CaCl_2$ to a sample of plasma. This test is widely used and the normal clotting times obtained under standard conditions range from about 12-14 seconds.

A major determinant of this clotting time is the level and activity state of factor VII. However, factor VII is present in high concentration in plasma relative to the amount of tissue factor added. Thus, normal clotting times (12 to 14 seconds) are obtained even when factor VII is higher than normal. The One Stage Prothrombin Time, however, is not sensitive to higher than normal levels of factor VII.

Although many aspects of the coagulation cascade have been elucidated, the initiation of clotting in vivo is still not fully understood. Specifically, the role of tissue factor has not been thoroughly investigated, since its role in both the intrinsic and extrinsic pathways of coagulation has only recently been recognized. See, e.g. Nemerson et al., Prog. Hemostasis & Thrombosis 6:237-261, 1982 and Nemerson, Blood 71:1-8, 1988. In addition, efforts to elucidate the mechanism of tissue factor activity in vitro have been hampered by difficulty in obtaining adequate amounts of pure protein for study.

Tissue factor has been purified to homogeneity from bovine brain in order to facilitate a detailed kinetic analysis of the reactions catalyzed by factor VII and tissue factor. Bach et al., J. Biol. Chem. 256:8324-8331, 1981. Classical purification techniques used to isolate the protein, such as immunoaffinity chromatography, even with the use of monoclonal antibodies to bovine tissue factor, are laborious and yield inadequate amounts of protein for detailed experiments and clinical use.

Likewise, small amounts of human tissue factor have been purified from human brains obtained from autopsy material and from term placentas. Broze et al., J. Biol. Chem. 260:10917-10920, 1985: Guha et al., proc. Natl. Acad. Sci. 83:299-302, 1986. The purified human tissue factor appeared to be a single polypeptide chain which had a molecular weight of 46,000 when analyzed by polyacrylamide gel electrophoresis in sodium dodecyl sulfate (SDS-PAGE) under both reducing and non-reducing conditions. The human protein, like bovine brain tissue factor, is resistant to cleavage by both trypsin and chymotrypsin. Immunologic studies have shown, however, that although bovine and human tissue factors function similarly, there is little cross-reactivity between the proteins from the two species.

Cloning the DNA sequences coding for the entire human tissue factor apoprotein would allow a determination of both the genetic and protein sequence for this biologically important protein. It is known that the gene coding for human tissue factor is located on chromosome 1. In addition to providing information about the DNA sequence coding for tissue factor and the amino acid sequence of the protein encoded therefrom, important information about the structure of the chromosomal gene can be obtained once the DNA coding for tissue factor is obtained. In addition, expression of the cloned human tissue factor gene in a suitable host would provide a ready source of tissue factor for clinical and diagnostic use as well as for experimental study. The DNA sequences encoding the proteins involved in tissue factor-initiated clotting (i.e., the extrinsic coagulation pathway), with the exception of tissue factor itself, have already been cloned and sequenced.

Recent reports have described the cloning of cDNAs coding for portions of human tissue factor. Morrissey et al., 1987, Fed. Proc. 46:716 (Abstr.); Scarpati et al., 1987, Fed. Proc. 46:2242 (Abstr.). Cloned DNA fragments coding for the entire tissue factor protein were not obtained in these studies. However, after the filing date of U.S. Patent Application Serial No. 062,166 which disclosed the cloning and sequence of the cDNA clone coding for the entire human tissue factor apoprotein, several publications reported the isolation and sequence of cDNA clones coding for human tissue factor. See, e.g. Morrissey et al., Cell 50:129-135, 1987; Spicer et al., Proc. Nat. Acad. Sci. 84:5148-5152, 1987; Scarpati et al., Biochemistry 26:5234-5238 1987; Fischer et al., Blood 48:89-99, 1987.

The present invention provides a complete, single cloned cDNA, the sequence of which codes for the entire human tissue factor apoprotein, the tissue factor apoprotein or a functional portion thereof encoded by the cloned cDNA and recombinant vectors containing the cloned cDNA which code for and can be induced to express the entire human tissue factor apoprotein, soluble human tissue factor protein, a truncated human tissue factor protein and functional portions thereof in suitable hosts.

## SUMMARY OF THE INVENTION

In accordance with the present invention replicable recombinant vectors are described which contain a cloned DNA, the sequence of which codes for the entire human tissue factor apoprotein and a functional portions thereof and also provide for the production of expression vectors which in suitable host cells express functional human tissue factor apoprotein, soluble tissue factor and truncated tissue factor. The recombinant vectors were derived by screening recombinant cloning vectors containing a human placental cDNA library for identifiable cDNA sequences coding for human tissue factor and isolating recombinant vectors containing cDNA coding for the entire human tissue factor apoprotein.

Suitable recombinant cloning vectors in which a cloned placental cDNA library can be made include those which replicate in prokaryotic hosts, such as bacteria, or those which replicate in eukaryotic hosts such as yeast, insect cells and animal or human cells. Suitable prokaryotic vectors include, but are not limited to, plasmids, cosmids and bacteriophage. Suitable eukaryotic vectors include inter alia vaccinia virus, bovine papilloma virus, simian virus$_{40}$ (SV$_{40}$) and baculovirus. It will be readily apparent to those skilled in the art that a wide variety of cloning vectors and hosts can be used in the practice of the invention.

The invention further provides a 2147 base pair (bp) cDNA which codes for the entire human tissue factor apoprotein, the sequence of such cDNA, and the human tissue factor protein and functional portions thereof encoded by the cDNA. The cDNA was identified and isolated from a cloned human placental cDNA library. The mature tissue factor apoprotein, a single polypeptide chain of 263 amino acids, is coded for by an open reading frame (ORF) of the cDNA fragment encompassing nucleotides 112 to 997. The actual translation product of the mRNA corresponding to the open reading frame is a preprotein of 295 amino acids having a leader sequence

3

or signal peptide of 32 amino which is cleaved postranslationally to form the mature human tissue factor apoprotein.

Furthermore, a 1.25 kb fragment of the 2157 bp cDNA has been isolated which includes nucleotides 90 through 1340 as provided in Formula I. This fragment, which contains the entire open reading frame and 5'- and 3'- flanking sequences was used to localize the tissue factor gene to a 9.5 kb DNA fragment from restriction enzyme digested total human genomic DNA (placental) and to produce recombinant expression vectors which in suitable hosts express the human tissue factor apoprotein and functional portions thereof, including soluble human tissue factor.

The invention further provides recombinant expression vectors which in a suitable transformant host provide for expression of the functional human tissue factor. Suitable expression vectors include those which replicate and express a desired protein in prokaryotic hosts, such as bacteria, or those which replicate in eukaryotic hosts such as yeast, insect cells and animal or human cells. Suitable prokaryotic vectors include, but are not limited to, plasmids, cosmids and bacteriophage. Suitable eukaryotic vectors include inter alia vaccinia virus, bovine papilloma virus, simian virus$_{40}$ (SV$_{40}$) and baculovirus. It will be readily apparent to those skilled in the art that a wide variety of cloning vectors and hosts can be used in the practice of the invention.

Of particular importance, such vectors have also allowed for production of a soluble form of the tissue factor protein which is missing the carboxy-terminal hydrophobic membrane spanning-portion of the protein. In particular, the vectors provide for expression of a soluble active tissue factor comprising the extracellular domain or the approximately N-terminal 219/220 amino acids of the mature human tissue factor apoprotein and a truncated human tissue factor comprising the 1-227 N-terminal amino acids of the mature human tissue factor apoprotein. Such soluble human tissue factor and truncated human tissue factor proteins and functional portions thereof, i.e., peptides derived from the extracellular domain of human tissue factor are especially useful as diagnostic reagents and anticoagulant agents.

## BRIEF DESCRIPTION OF THE DRAWINGS

The present invention is now described with reference to the detailed description, Examples and figures in which

Fig. 1 is a representation of the amino acid sequence of the mature human tissue factor apoprotein and hydropathy plot thereof.

Fig. 2 provides the nucleotide sequence of human tissue factor-specific oligonucleotide probes used to screen a cloned placental cDNA library.

Fig. 3 is a schematic representation of the 2147 bp cDNA which codes for human tissue factor apoprotein and the strategy used to obtain the nucleotide sequence thereof.

Fig. 4 shows the construction of recombinant plasmid pKS-2B and recombinant phage m13/LB2TF.

Fig. 5A shows the construction of recombinant plasmid pLB4TF.

Fig. 5B shows the construction of M13/TL131P.

Fig. 5C provides the sequence of oligonucleotides TFAD and G8PST used for site specific mutagenesis.

Fig. 6 shows the construction of the human tissue factor expression plasmid pTL8FQ.

Fig. 7 depicts the pMAM/TF shuttle vector for expressing human tissue factor in mammalian cells.

Fig. 8 is a schematic representation of the construction of plasmid pLB5TF.

Fig. 9 is a schematic representation of the construction of the soluble tissue factor expression vector pLB6TF.

Fig. 10 shows the construction of M13/LB2TF.

Fig. 11 shows the construction of pLB6TF.

Fig. 12 shows the construction of pTL8TFA-I wherein S = gene VIII (M13) signal peptide E = extracellular domain and M = membrane domain.

Fig. 13 shows the activity of the truncated human tissue factor toward Factor VII.

## DESCRIPTION OF THE INVENTION

The present invention provides for replicable recombinant vectors containing a cloned cDNA insert, the sequence of which codes for and, in a suitable host, express the entire human tissue factor apoprotein or functional portions thereof. The recombinant vectors have been obtained by screening for DNA sequences coding for human tissue factor in a cloned human placental cDNA library which had been inserted into a cloning vector replicable in a suitable host. Preferred vectors for cloning the human cDNA library include plasmids, bacteriophage and other vectors replicable in bacteria, and vectors replicable in eukaryotic hosts such as yeast, insect cells and animal or human cells. Prokaryotic vectors include inter alia plasmids, cosmids and bacteriophage. Eukaryotic vectors include inter alia vaccinia virus, bovine papilloma virus, SV$_{40}$, yeast vectors and baculovirus. A preferred cloning vector is bacteriophage λgt11, an expression vector described by Young and Davis, Science 222:778-782, 1983. A preferred host organism is E. coli strain K1088, a known host for λgt11.

4

The 2147 bp DNA fragment encoding the entire human tissue factor apoprotein was isolated from a human placental cDNA library cloned into λgt11. Human tissue factor coding sequences were identified in the cloned placental cDNA library by screening recombinant bacteriophages whose DNA hybridized to specific oliognucleotide hybridization probes. The DNA sequences of the hybridization probes correspond to and code for amino acid sequences of short peptide fragments from (i) the amino terminal, (ii) the carboxy terminal and (iii) an internal portion of the tissue factor polypeptide chain.

The present invention, in particular, provides a recombinant bacteriophage λ10,3 which was derived from the placental cDNA library cloned in phage λgt11 and contains the cloned 2147 bp cDNA insert which codes for the entire human tissue factor apoprotein obtained from the placental cDNA library. Also encompassed by the invention is a second bacteriophage, λ3,4 which was also derived from λgt11 and contains tissue factor coding sequences. Phage λ3,4 contains a cloned 1616 bp cDNA insert from the human placental bp cDNA library, the nucleotide sequence of which is identical to the 3′ portion of the 2147 bp DNA insert of phage λ10,3 and codes for the carboxy-terminal portion of the tissue factor apoprotein.

The invention also provides for the cloned 2147 bp cDNA coding for the entire human tissue factor apoprotein, the sequence of which is provided in Formula I. The sequence of the DNA is characterized by a single open reading frame extending from an ATG initiation codon at nucleotides 112-114 to a TAA termination codon at nucleotides 997-999 which encodes a single polypeptide chain of 295 amino acids, which is the tissue factor preprotein containing a leader sequence of 32 amino acids. The mature tissue factor apoprotein is a single polypeptide of 263 amino acids whose amino terminal sequence is Ser-Gly-Thr-Thr-Asn. The preprotein whose sequence is also provided in Formula I (beginning at residue -32) is postranslationally converted to the mature tissue factor apoprotein whose sequence begins at the amino acid residue marked +1 as shown in Formula I. The molecular weight of the mature tissue factor apoprotein (without carbohydrate) was calculated to be approximately 29,600.

The invention also provides for a recombinant plasmid, pKS-2B, which was derived from the E. coli cloning vector pUC19 by inserting an approximately 4.15 kb DNA fragment obtained from λ10,3 which comprises the 2147 cDNA insert coding for tissue factor plus about 1000 bp of λDNA flanking each end of the tissue factor cDNA insert into the lacZ′ gene of pUC19. pKS-2B has been used to transform E. coli strain 71-18 which is a known host for pUC19. E. coli 71-18/pKS-2B transformants are a preferred source of the 2147 bp cDNA fragment coding for human tissue factor.

EP 0 347 262 A1

Formula I

1   CGGGCGAACGGGCTGGCACTGCCTCTGCGGGGGGGCCAGGGGGGCTTCAGGGGCAAGCTGCCCAGGGGCAGGGGGGGCAGGGAAGGGGGCTGGATCTGCGGGGGCAACTGGTAGACATGGAGAGCC
                                                                                                                          -32 MetGluThr

121  CCTGGCTGCGGGGGGGGTGGGGGGGGCGGGGAGAGGGGGGTGGCTGGCAGGCTGCTGCTGCGGGGCTGGGGTCTTCGGGGCAGGTGGGGGGGGGCTTCAGGCACTACAAATACTGTGGCCAGCCATATAAT
-29  ProAlaTrpProArgValProArgProGluThrAlaValAlaArgThrLeuLeuLeuGlyTrpValPheAlaGlnValAlaGlyAlaSerGlyThrThrAsnThrValAlaAlaTyrAsn

241  TTAACTTGGAAATCAACTAATTTCAAGACAATTTTGGAGTCGGAACCCAAACCCGTCAATCAAGTCTACACTGTTCAAATAACCACTAAGTCAGGAGATTCGAAAAGCAAATCCTTTTAC
12   LeuThrTrpLysSerThrAsnPheLysThrIleLeuGluTrpGluProLysProValAsnGlnValTyrThrValGlnIleSerThrLysSerGlyAspTrpLysSerLysCysPheTyr

361  ACAACAGACACACAGAGTGTGAGCTCAGGGGAGGAGCATTGTCAAGGCATGTGAAGCAGAGGTACTTCGGCAGGGGTCTTCTCCTACGGGGGGCAGGGAATGTGGAGAGCAGGGGTTCTGCTGGGGGAG
52   ThrThrAspThrGluCysAspLeuThrArgGluIleValLysAspValLysGlnThrTyrLeuAlaArgValPheSerTyrProAlaGlyAsnValGluSerThrGlySerAlaGlyGlu

481  CCTCTGTATCAGAACTCCCCAGAGTTCACACCCTTACCTCGAGACAAACCTCCCACACAGGCAACAATTCAGAGTTTTCAACACGGTCGGCAACAAAAGTGAATGTGACCGTAGAACATGAACCG
92   ProLeuTyrGluAsnSerProGluPheThrProTyrLeuGluThrAsnLeuGlyGlnProThrIleGlnSerPheGluGlnValGlyThrLysValAsnValThrValGluAspGluArg

601  ACTTTAGTCAGAAGCAACAACACTTTCCTAAGCCTGGGGCATGTTTTTGGCAAGGACTTAATTTATACACTTTATTATTGGAAATCTTCAAGTTCAGGAAACAAAACAGGCAAAACAAAC
132  ThrLeuValArgArgAsnAsnThrPheLeuSerLeuArgAspValPheGlyLysAspLeuIleTyrThrLeuTyrTyrTrpLysSerSerSerGlyLysLysThrAlaLysThrAsn

721  ACTAATCAGTTTTTCATTGATGTGGATAAAGGAGAAAACTACTGTTTCAGTGTTCAACCAGTCATTCCTCGGGGAACAGTTAACCGGAAGAGTACACACAGGGGGGTACAGTGTATGGGC
172  ThrAsnGlnPheLeuIleAspValAspLysGlyGluAsnTyrCysPheSerValGlnAlaValIleProSerArgThrValAsnArgLysSerThrAspSerProValGluCysAsnGly

841  CAGGAGAAACGGCAATTCAGAGAAATATTCTACATCATTGGACCTGTGTGGTATTGTGTGGTCATCATGCTTGTCATCATGCTGGCTATATCTCTACACAAGTGTAGAAAGGCCAGGAGTGGGG
212  GlnGluLysGlyGluPheArgGluIlePheTyrIleIleGlyAlaValValPheValValIleIleLeuValIleIleLeuAlaIleSerLeuHisLysCysArgLysAlaGlyValGly

961  CAGAGCTGGAAGGAGAACTCCCCACTGAATGTTTCATAAAGGAAGCACTGTTGCAGGCTACTGCAAATGCTATATTGGACTGTGACCGAGAACTTTTAAGAGGATAGAATACATGGAAACG
252  GlnSerTrpLysGluAsnSerProLeuAsnValSerEnd

1081 CAAATGAGTATTTGGGAGGCATGAAGAGGCTGGAGTTCAAAAAACTCTTGATATGAGCTGTTATTAGCATTAGCATTCTGGTTTTGACATCAGCATTAGTCACTTTGAAATGTAAGGAATG
1201 GTACTACAGGCAATTGCAAGTTTTAATTTTTAACAGCATGGCAGCTTTTGCACATAAGCATGCTTTAGATTATATATTGGGCACTTAAGGATTAAGCAGGTGGTGCAAGCAAAAACAAATG
1321 GGAAAATGTGTCTTAAAAAAATGCTGGGGTGGACTTTTTGAAAAGCTTTTTTTTTTTTTTTTTTTTTGAGAGGGAGTCTTGCTCTGTTGGGCAGGCTGGAGTGCAGTAGGCAGGATCTGGGCTGCACT
1441 TGCACGCTGGGTGTCTGGGGTTCAAGGCAATTGTGTGGGCTCAGGCTGGGGAGTAGGCTGGGATTACAGGTGGGGCACTAGCAGGGGCAAGGCTAATTTTTTGTATTTTTTTAGTAGAGATGGGGTTT
1561 CAGGGATGTTGGGGCAGGGTGGGTGTGTCTTGAATTGCTGAGGCTGAGTGATCGAGGGGACGGTTGGGGCTGGGGAAAGGATGCTAGTATTATGGGGGGTGAAGCAGGGATGGGGCAGGGGGAAAAGGCTTTTGAGGG
1681 GGTGCACTTCAATGCATGTAGGGAAAGTAAAATGGAAGGGAAATTGGGGTGGCATTTCTAGGGACTTTTTCTAACATATGTCTATAATATAGTGTTTAGGGTTCTTTTTTTTTTTTTCAGGCAATACATTTG
1801 GAAATTCAAAACAATTGGGCAAACTTTGTATTAATGTGTTAAGTGCAGGGAGACATTGGTATTCTGGGGCAGGCTTGCTAATATGCTTTTACAATCTGCACTTTTAAGCTGACTTTAAAGTGGGCATTA
1921 AACATTTCGAGAGCTAACTATATATTTTTTATAAGACTACTATACAAACTACAGAGTTTTATGATTTTAAGGGTACTTAAAGGCTTTCTATGGGTTTGACATTTGTATATATATAATTTTTTTAAAAAAAGGCTTTTTT
2041 CTATATGGGGGATTTTTCTATTTTATGTAGGGTAATATTGTTTCTATTTGTATATATATTGAGATAAATTTATTTAATATACTTTAAAATAAAGGGTGACTGGGGAATTGTTAAAAAA

The availability of the cloned 2147 bp cDNA coding for tissue factor has allowed characterization of the human genomic gene which codes for the protein. A 1.25 kb DNA fragment encompassing nucleotides 90-1340 shown in Formula I which comprises the open reading frame and some 5'- and 3'-flanking sequences has been obtained by restriction enzyme digest of plasmid pKS-2B. The 1.25 kb fragment hybridized to a 9.5 kb DNA fragment of digested human placental genomic DNA. This 9.5 kb genomic DNA fragment is believed to contain at least 3 introns within the sequence coding for tissue factor. Although the tissue factor gene has been mapped to human chromosome 1, the gene has not heretofore been localized, to a specific genomic DNA fragment. The 1.25kb fragment has also been used to construct expression vectors for production of the entire human tissue factor apoprotein and soluble human tissue factor in suitable host cells.

Based on the amino acid sequence of the entire human tissue factor apoprotein predicted from the sequence of the cDNA insert of $\lambda$10,3 encoding the protein and confirmed by amino acid sequence analysis of about 70% of the protein, a domain structure is proposed for this integral membrane bound protein. A protein domain can be defined as an independently folded functional region of a protein. Each of the domains of the human tissue factor apoprotein provided by the present invention has unique structural and functional features: (1) a signal peptide or leader sequence region of 32 amino acids which is post translationally removed upon conversion of the preprotein to mature active tissue factor; (2) an extracellular, generally hydrophilic, N-glycosylated domain which comprises the approximately amino terminal 219 amino acids; (3) an approximately 23 amino acid stretch of mainly hydrophobic amino acids comprising approximately amino acids 220 to 242 which is believed to be that portion of the protein which spans the cellular membrane; and (4) the carboxy terminal approximately 21 amino acids comprising approximately amino acid residues 243 to 263 which is believed to be the cytoplasmic domain on the inside of the cell.

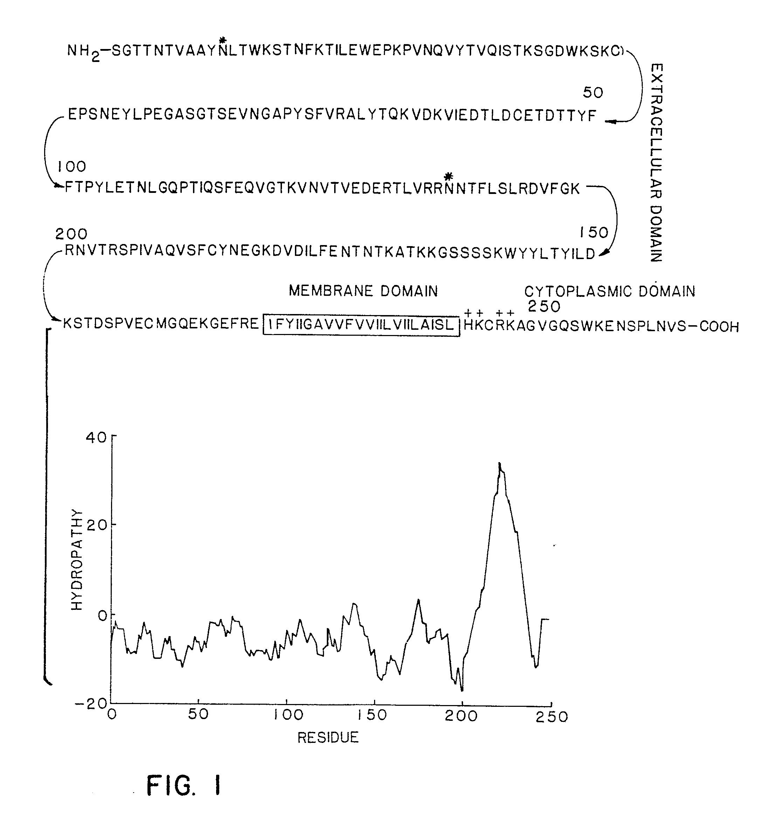

Fig. 1 represents what is believed to be the domain structure of the substantially pure mature human tissue factor apoprotein of the invention (from which the 32 amino acid signal peptide is removed), the amino acid sequence of which is provided in Formula I. The several salient features of the proposed domain structure (1-4) of tissue factor can be seen with reference to Fig. 1.

Four potential N-linked carbohydrate attachment sites (Asn-X-Ser/Thr) are found in the molecule. One of these sites occurs in the cytoplasmic domain at the carboxy terminus and therefore is probably not glycosylated. Of the three sites in the extracellular domain, each of which is marked by a ( $\phi$ ) in Fig. 1., two have been identified as bearing carbohydrate by amino acid sequence analysis. As discussed in Example 1 no PTH amino acid could be identified at amino acids 11 and 137 (Formula I) which were predicted from the cDNA sequence to be asparagine. Each of these Asn residues occupies the beginning of a carbohydrate attachment site. In general, carbohydrates are only found on the extracellular protein of membrane-bound glycoproteins like tissue factor, so it would be expected that the carbohydrate would only be found on the extracellular domain.

It is also apparent with reference to Fig. 1 and Formula I that human tissue factor contains a total of five half-cystine residues (circled in Fig. 1), four of which occur within the extracellular domain and are probably disulfide linked. The presence of disulfide bridges had been inferred previously by an observation by Bach et al., J. Biol. Chem. 256:8324-8331, 1981, for bovine tissue factor that reduction with 2-mercaptoethanol in the presence of SDS resulted in loss of tissue factor procoagulant activity, whereas treatment with SDS alone did not. It is assumed that Cys at position 245 of human tissue factor is not involved in intramolecular disulfide bond formation since it would be expected to be segregated on the cytoplasmic domain. Further confirmation that Cys[245] of human tissue factor is not involved in intramolecular disulfide bond formation was provided by the observation that CNBr cleavage at the single methionine residue, Met[210], liberated a carboxy-terminal peptide from purified tissue factor without disulfide bond reduction. Cys[245] may form intermolecular disulfide linkages, perhaps modulating tissue factor self-association, as described by Bach et al., Biochemistry 25:4007-4020, 1986, or by interaction with other substances such as proteins or fatty acids during purification.

Also shown in Fig. 1 is a hydropathy plot determined by the method of Kyte and Doolittle, J. Molec. Biol. 157:105-132, 1982, which graphically depicts the hydrophobic character of the membrane domain of tissue factor. Each value was calculated as the average hydropathic index of a sequence of 21 amino acids and plotted to the middle residue of each sequence. The consecutive stretch of 23 non-polar amino acids at the carboxy terminal region of the protein is characteristic of the domains of integral membrane proteins which span the lipid bilayer of the plasma membrane of cells. In addition, four positively charged amino acids (marked as + in Fig. 1) are observed immediately adjacent to the carboxy side of the hydrophobic region, which is a characteristic feature seen at the interface between membrane and cytoplasmic domains of many integral membrane proteins. (See, e.g., Sabatini et al., J. Cell. Biol. 92:1-21, 1982.) Furthermore, the presumed cytoplasmic domain of tissue factor has features common to both the low density lipoprotein receptor and thrombomodulin in that a comparison of this region in all three proteins indicates that the cytoplasmic domain is short and contains a single Cys residue. (See, e.g., Jackman et al., Proc. Natl. Acad. Sci. 83:8834-8838, 1986 and Yamamoto et al., Cell 39:27-38, 1984).

The defined sequence of the substantially pure human tissue factor as provided in Formula I and its apparent domain structure has allowed the production of a truncated human tissue factor and a soluble human tissue factor which comprises the extracellular domain of the protein or portions thereof which is exportable from transformed hosts containing expression vectors which express soluble tissue factor. The sequence also allows for the production of functional portions of human tissue factor, i.e., peptides derived from human tissue factor, which can be used as diagnostic reagents and to inhibit the binding of Factor VII to tissue factor.

Because intact tissue factor is a membrane-bound hydrophobic protein, in general it is not readily secretable from transformed hosts containing vectors for expression of the entire human tissue factor. The availability of large amounts of the soluble form of tissue factor, however, allows for production of the intact protein. The membrane spanning and cytoplasmic domains of human tissue factor can be produced by suitable techniques, e.g. solid phase peptide synthesis. The intact human tissue factor apoprotein is reconstitutable by covalently attaching the soluble human tissue factor produced by recombinant DNA techniques or other methods with the membrane/cytoplasmic domain(s) produced by peptide synthetic techniques through protein synthetic methods such as fragment condensation or protein semi-synthesis techniques.

The soluble tissue factor of this invention is lacking the hydrophobic membrane-spanning and cytoplasmic domains. Surprisingly, such soluble tissue factor, which has not been previously produced, has some minimal procoagulant activity as measured by the activation of Factor VII and the subsequent cleavage of Factors IX and X in standard two stage clotting assays. The soluble tissue factor, which efficiently binds to Factor VII, also has use as an inhibitor of the procoagulant activity of native human tissue factor. Soluble tissue factor, thus, has great potential importance as a diagnostic reagent as well as a potential anticoagulant agent for clinical use.

The cloned 2147 bp cDNA coding for human tissue factor and plasmid pKS-2B have allowed the construction of recombinant expression vectors which in transformed hosts produce human tissue factor apoprotein, soluble human tissue factor and truncated human tissue factor for clinical and diagnostic use, as well as experimental studies. Such expression vectors include those which replicate and express tissue factor in prokaryotic hosts, such as bacteria, or those which replicate in eukaryotic hosts such as yeast, insect cells and animal or human cells. Suitable prokaryotic vectors include, but are not limited to, plasmids, cosmids and bacteriophage. Suitable eukaryotic vectors include inter alia vaccinia virus, bovine papilloma virus, simian virus$_{40}$ (SV$_{40}$), yeast vectors, and baculovirus. It will be readily apparent to those skilled in the art that a wide variety of cloning vectors and hosts can be used in the practice of the invention.

In particular, soluble tissue factor encompassing amino acids approximately 1-219/220, i.e. the extracellular domain of the mature apoprotein, has been prepared by DNA cloning techniques. The DNA coding for soluble active tissue factor was prepared by inserting a stop codon in the tissue factor gene immediately downstream from the nucleotides coding for amino acids 219/220 of the tissue factor protein sequence provided in Formula I. This resulted in deletion of the hydrophobic membrane spanning region (domain 3) as well as the cytoplasmic tail (domain 4), of human tissue factor. (See Fig. 1).

Alternatively, soluble human tissue factor comprising the extracellular domain, with or without the signal peptide, may be generated by other techniques including site specific mutagenesis to produce a soluble protein or by cleavage of the mature protein (without the signal peptide) encoded by the 2147 bp cDNA insert in λ10,11 with CNBr which cleaves the protein at the unique Met residue at position 210 of the mature protein. Such soluble tissue factor proteins have use as anticoagulants and as diagnostics for clotting disorders. Furthermore, functional portions of the soluble proteins corresponding to peptides derived from the soluble protein can also be used as anticoagulants and as diagnostic reagents.

Intact human tissue factor apoprotein, having procoagulant activity, may be reconstituted from the soluble tissue factor by addition thereto of amino acids thereto which comprise the hydrophobic and cytoplasmic domains of tissue factor. The hydrophobic and cytoplasmic domains may be added to the soluble tissue factor protein by known protein chemistry techniques, such as fragment condensation and protein semi-synthesis.

Truncated human tissue factor encompassing amino acids 1-227 of Formula 1 has also been prepared by construction of an expression vector containing the human tissue factor gene with a 110 base pair deletion from the 3' end of the cDNA coding sequence of Figure 1.

The invention also provides for those portions of the human tissue factor and the DNA sequences encoding each portion of the protein which have the structural and functional characteristics of the domains (1)-(4). The invention further provides for the substantially pure soluble tissue factor and truncated tissue factor which have not been previously produced.

E. coli strain 71-18 carrying plasmid pKS-2B and pTL8TFA-I have been deposited with the American Type Culture Collection and assigned Accession No. 67426 and    and are available from Dr. Konigsberg's laboratory at Yale University.

The following examples are provided to illustrate the invention and are not intended to limit the same.

Example 1

Amino Acid Sequence Analysis of Purified Human Placental Tissue Factor

Approximately 67% of the amino acid sequence of human placental tissue factor was obtained by protein sequencing techniques prior to obtaining the cloned cDNA coding for tissue factor. A knowledge of the amino acid sequence allowed for the design of the specific oligonucleotide hybridization probes described in Example 2 which were used to screen for DNA sequences which coded for tissue factor in the cloned placental cDNA library. The protein sequencing was performed as follows:

A monoclonal IgG, antibody, HTF1-7B8, against human brain tissue factor that had been purified using factor VII affinity columns as described by Guha et al., 1986, Proc. Natl. Acad. Sci. 83:299-302, was prepared by Carson et al., Blood 70:490-493, 1987. This antibody, which was obtained from Dr. Steven D. Carson of the University of Colorado Health Sciences Center and Dr. Ronald Bach of the Mount Sinai School of Medicine, was used to prepare an immunoadsorbent column for immunoaffinity isolation of human tissue factor. Tissue factor was extracted from human brain or placental tissue acetone powders with the detergent octylphenoxy polyethoxy (10) ethanol (Triton® X-100) and then adsorbed onto the immunoaffinity column. Following washing with Triton® X-100-containing buffers at pH 7.5, the protein was eluted from the column at pH 2.5. It was then concentrated and further purified on an Ultragel AcA 34 column in Triton® X-100, which separated tissue factor from co-eluting minor contaminants to yield a substantially homogeneous protein preparation. The purity of each preparation was assessed by SDS-PAGE (Laemmli, Nature 227:680-685, 1970). The purification was monitored by measuring the tissue factor procoagulant activity in the preparation by a standard two-stage coagulation assay following reconstitution of the detergent-solubilized protein into phospholipid vesicles as described by Bach et al., J. Biol. Chem. 256:8324-8331, 1981.

The amino acid composition and protein concentration of each tissue factor preparation was determined by amino acid analysis: 10 μg of human tissue factor apoprotein in 0.1M NaCl, 0.05M Tris (pH 7.5), and 0.1% Triton® X-100 were precipitated by the addition of 10% trichloroacetic acid (TCA). After 15 minutes on ice the protein was pelleted by centrifugation at 5,000xg for 30 minutes. Residual detergent and TCA were removed by acetone extraction (3x). The pellet was dried in vacuo, and hydrolyzed in 6N HCl containing 0.2% phenol for 6 hours at 115°C. Analysis of the hydrolysate was performed on a Beckman 121M amino acid analyzer.

Amino acid sequence analysis of intact tissue factor and peptide fragments generated therefrom was performed using an Applied Biosystems (Foster City, CA) gas-phase sequenator. The phenylthiohydantoin (PTH) derivatives liberated at each cycle of sequencing were identified as described by Merrill et al., J. Biol. Chem. 259:10850-10856, 1984. The amino-terminal sequence of intact brain and placental tissue factor was determined using 100-200μg of each apoprotein. The protein was precipitated as described above, dissolved in 100% trifluoroacetic acid (TFA) and applied to a GF/C glass filter disc for gas-phase sequencing. Following TCA precipitation and acetone extraction the protein was dissolved in 0.8ml 6M guanidine HCl, 50mM NaHCO3, pH 8.0. The amino-terminus of the protein was blocked by succinylation with three successive additions of 1mg solid succinic anhydride (Pierce Chemical Co.). After each addition the sample was adjusted to pH 8.0 with 1N NaOH and stirred for 30 minutes. Triton® X-100 was then added to a final concentration of 0.1% and the mixture was dialyzed overnight at 25°C. Following addition of 450μl H2O, the digest was dried (2x) in a vacuum centrifuge. The protein pellet was dissolved in TFA, loaded onto a GF/C glass filter disc and sequenced as described above.

A partial amino acid sequence, spanning residues 211-244, of the carboxy-terminal CNBr peptide (residues 211-263), was obtained by succinylating the intact protein and cleaving at the single methionine residue in the protein, Met[210], with CNBr, followed by gas-phase sequence analysis. The carboxy-terminal CNBr peptide was prepared from about 60 μg of the placental apoprotein.

Tryptic peptides of TCA-precipitated placental tissue factor (120μg) were also prepared. After isolation by HPLC, the peptides were sequenced in order to obtain additional sequence information. The TCA-precipitated protein pellet was solubilized in 50μl 8M urea and the solution was diluted with 150μl 50mM NH4HCO3. Trypsin, treated with N-tosyl-L-phenylalanine chloromethyl ketone (TPCK, Cooper Biologicals), was then added at a ratio of 1:25(w/w), trypsin:tissue factor. Digestion was carried out for 24 hours at 37°C and the sample was injected into a Vydac® C-18 HPLC column (0.45x25cm) equilibrated in 0.05% TFA and run at a flow rate of 1ml/min. Peptides were eluted with linear gradients of buffer B (0.05% TFA, 80% acetonitrile) as follows: 0-63 min. (2%-37.5% B), 63-95 min (37.5%-75% B), and 95-105 min (75%-98% B). The elution profile was monitored by absorbance at 210nm and 280nm. The isolated tryptic peptides were sequenced as described above for the intact protein.

## Example 2

### Isolation of Phages λ10,3 and λ3,4

Phage λ10,3 which contains the 2147 bp cDNA insert coding for human tissue factor was isolated by screening a human placental cDNA library cloned into the expression vector phage λgt11 for human tissue factor coding sequences. The library, which contained about 1.2 x 10⁶ independent placental cDNA-containing recombinants, was purchased from CloneTech (Palo Alto, CA). λgt11 is a well-known cloning vector (Young and Davis, Science 222:180-182, 1983) which contains the E. coli lacZ gene coding for β-galactosidase. Foreign DNA can be cloned into the EcoRI restriction site of the lacZ gene.

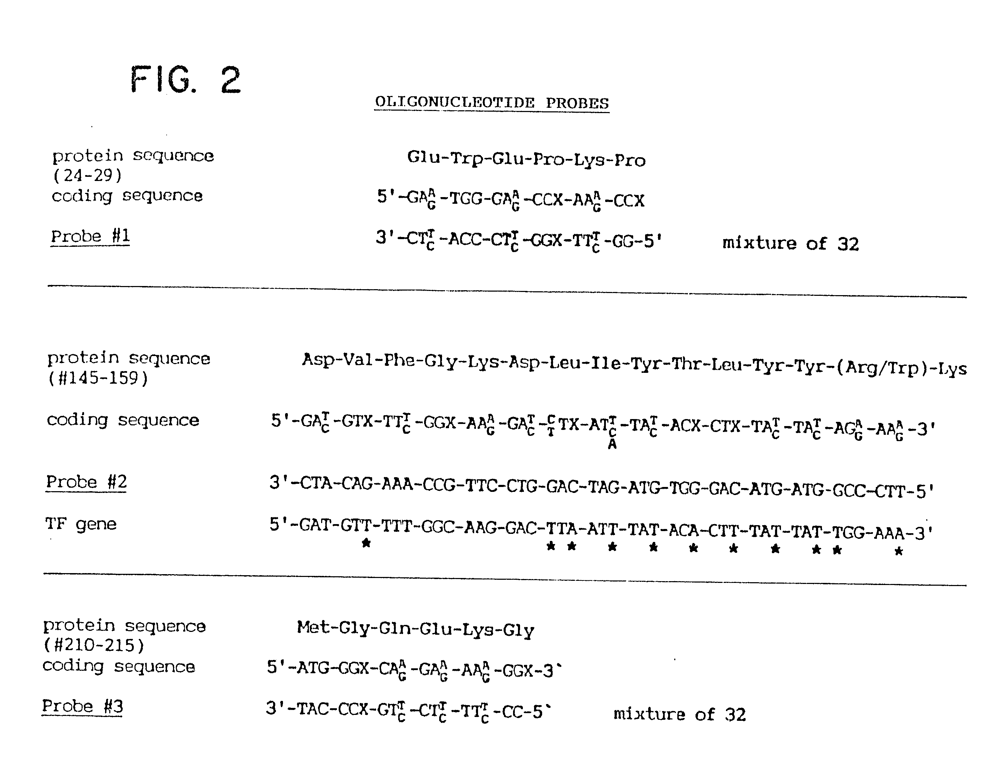

To screen for tissue factor coding sequences in the cloned placental cDNA library, three oligonucleotide hybridization probes were synthesized. These probes which are shown in Fig. 2 hybridized to DNA corresponding to and coding for amino acid sequences of short peptides from various regions (amino terminal, carboxy terminal and internal) of the tissue factor polypeptide chain which were determined as provided in Example 1. The oligonucleotide probes were synthesized by the phosphoramidite method, using an Applied Biosystems 380A DNA synthesizer. The probes were purified by HPLC on a Nucleogen DEAE 60-7 column (Macherey-Nagel) and radiolabelled at their 5′ ends using $^{32}$P ATP (Amersham) and T4 polynucleotide kinase (Pharmacia) to a specific activity of approximately 1 x $10^8$ cpm/μg.

The determination of the partial amino acid sequence of human tissue factor, as provided in Example 1, allowed the construction of the oligonucleotide hybridization probes. With reference to Fig. 2 it can be seen that the probes correspond to DNA coding for three regions of the tissue factor protein. Probe #1 corresponded to the sequence coding for amino acids 24-29 (amino terminal portion of the protein); Probe #3 corresponded to the sequence coding for amino acids 210-215 (carboxy terminal portion of the protein); and probe #2 corresponded to the sequence coding for amino acids 145-149 (internal portion of the polypeptide chain). Probes #1 and 3 were both mixtures of 32 oligonucleotides, each 17 bases in length, which were complementary to all the possible coding sequences for each peptide. Probe #2 was a single 45 base deoxyoligonucleotide (45mer) corresponding to an optimal DNA sequence coding for an internal trypic peptide of human tissue factor. Selection of the optimal coding sequence for the 45mer probe was based on the codon preference in human structural genes described by Lathe, J. Molec. Biol. 183: 1-12, 1985.

Fig. 2 also depicts that portion of the sequence of the cDNA coding for human tissue factor provided in Formula I in the region of the cDNA fragment which hybridized to Probe #2. The asterisks (*) indicate base pair mismatches between Probe #2 and the actual tissue factor cDNA coding sequence as determined in Example 3. The overall homology between Probe #2 and the cDNA coding for tissue factor was 75%. Residue 158, originally identified as Arg was subsequently identified as Trp.

The placental cDNA library was screened by plating the recombinant phage on a lawn of E. coli K1088 cells grown on agar plates. E. coli K1088, the host for λgt11, carries the plasmid pMC9 which confers ampicillin resistance and carries the lacI gene (for efficient repression of the lacZ gene). See, Young and Davis, Science 222:778-782, 1983. Screening was performed essentially according to the protocol of Maniatis et al., Molecular Cloning: A Laboratory Manual, Cold Spring Harbor Laboratory, Cold Spring, New York, 1982, by transferring recombinant λgt11 plaques from the agar plates to nitrocellulose filters and hybridizing immobilized phage DNA to the ($^{32}$P)-oligonucleotide probes.

Approximately 3-5 x $10^4$ phage per 85 cm plate which had plated on E. coli K1088 were screened by duplicate filter lifts (Colony/Plaque Screen, New England Nuclear) to the probes. Plaques were first screened by hybridization to Probe #2 at $T_m$-25°C (50°C) in 6X SSC (1X SSC = 0.15 M NaCl, 0.015 M Na$_3$ citrate, 0.1% sodium dodecyl sulfate (SDS). The filters were then washed at $T_m$-8°C in 2X SSC and autoradiographed at -70°C with an intensifying screen (DuPont Lightening Plus) for 16 to 40 hours. Phage which gave positive signals with Probe #2 were subsequently plaque-purified and rescreened by hybridization to Probes #1 and #3 as described by Benton and Davis, Science 196:180-182, 1977, to eliminate false positives.

Approximately 2.5 x $10^6$ recombinant λgt11 phage which contained placental cDNA inserts were screened with Probe #2. Thirty-six potentially positive plaques were isolated from the library, purified and further screened by hybridization to Probes #1 and #3. In all cases Probe #2 gave significantly stronger hybridization signals than either Probe #1 or #3. Only two of the 36 original recombinant λ phage isolates were reactive with a second probe. The recombinant phage designed λ10,3 was positive with both Probes #1 and #3, while the recombinant phage designated λ3,4 only hybridized to Probe #3.

The number of recombinant phage clones containing a cDNA coding for human tissue factor was, therefore, estimated to be between 2 and 34 per 2.5 x $10^6$ recombinants. The relative abundance of tissue factor coding sequences in placental cDNA was thus estimated at one human tissue factor cDNA per 7 x $10^4$ to 1 x $10^6$ cDNAs.

The recombinant phage λ10,3 and λ3,4 have been successfully propagated in their host organism E. coli K1088. This has allowed the production of large quantity of recombinant phage λ10,3 and λ3,4 containing the DNA inserts coding for human tissue factor.

Example 3

Sequence and Characterization of the 2147 bp cDNA Coding for Human Tissue Factor

Restriction analysis showed that λ10,3 contained a 2147 bp DNA insert while λ3,4 contained a 1616 bp insert. EcoRI, Sau3A, and HindIII restriction fragments of λ10,3 and λ3,4 were cloned into the phage cloning vectors M13mp18 and M13mp19 (Messing, Meth. Enzymol. 101:20-78, 1983) to facilitate nucleotide sequence analysis. The sequence of the cDNA inserts in each of the two recombinant phage was determined by the dideoxy chain termination method of Sanger et al., Proc. Natl. Acad. Sci. 74:5463-5467, 1977, using ($^{35}$S)-deoxyadenosine 5′-(α-thio) triphosphate (Amersham; 500 mCi/mmole). Sequencing reactions were analyzed on 6% polyacrylamide-7M urea gels, which were dried overnight prior to autoradiography. Chain extension reactions were primed using M13 seventeen nucleotide primers which were complementary to positions -20 and -40 relative to the EcoRI site (New England Biolabs; primers #1211 and #1212). Furthermore, eight different 18mer oligonucleotides complementary to human tissue factor cDNA coding sequences were synthesized as described in Example 2 and used to initiate sequencing reactions within large inserts in M13.

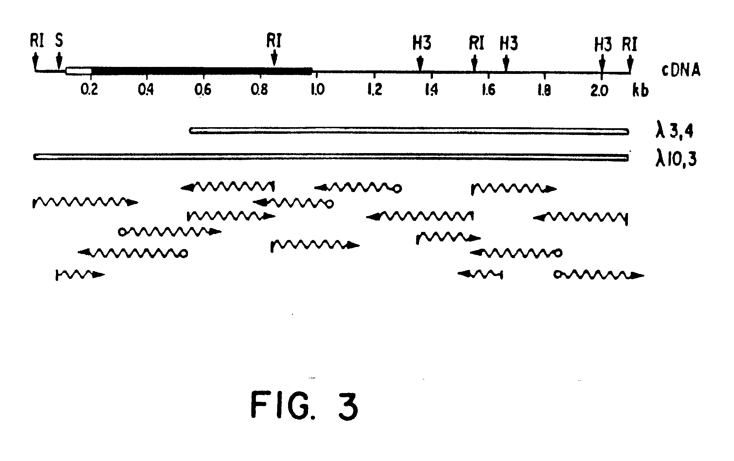

Fig. 3 shows a restriction map of the entire cloned 2147 bp cDNA coding for human tissue factor inserted in λ10,3 and also indicates the corresponding truncated 1616 bp DNA insert in λ3,4. Both of the cDNAs contained two internal EcoRI sites and the smaller cDNA of λ3,4 was found to overlap entirely with the larger DNA insert of λ10,3.

With reference to Fig. 3, the restriction sites used to generate the M13 subclones are indicated at the top of the figure (RI = EcoRI; S = Sau3A; H3 = HindIII). The open and solid boxes indicate the coding region for the signal peptide and mature protein, respectively. The second and third lines of Fig. 3 show the relative size and location of the tissue factor cDNA insert in λ3,4 and λ10,3, respectively. The wavy lines indicate the length and direction of the DNA sequence determined from the M13 subclones. The open circles indicate where the synthetic primers were used to originate sequencing reactions. These primers correspond to the sequence of the 2147 bp cDNA at nucleotides 283-267, 326-339, 539-527, 821-838, 1075-1039, 1310-1293, 1875-1857 and 1857-1875. The nucleotide sequence was determined for both strands of DNA for 100% of the cDNA coding for the mature tissue factor apoprotein and approximately 82% of the overall cDNA sequences.

The nucleotide sequence of the entire 2147 bp cDNA from λ10,3 coding for human tissue factor is provided in Formula I. With reference to Formula I, the 2147 bp cDNA contains a single long open reading frame of about 885 bp which extends from an ATG initiation codon at nucleotides 112-114 to a TAA termination codon at nucleotides 997-999. The open reading frame codes for a preprotein of 295 amino acids which is the precursor for the 263 amino acid mature human tissue factor apoprotein. The entire preprotein sequence which was deduced from the DNA sequence is also provided in Formula I. Based on a comparison to the amino terminal sequence of the mature human tissue factor (Ser-Gly-Thr-Thr-Asn) as determined in Example 1 it was seen that the preprotein contains a 32 amino acid leader sequence or signal peptide.

As shown in Formula I, the 5′ untranslated region of the mRNA corresponding to the 2147 bp cDNA is very GC rich (72% G + C) as are a number of other eukaryotic signal peptide regions (see, e.g., Ohno et al., Nature 325:161-166, 1987). The 3′ untranslated sequence of 1147 nucleotides following the coding portion was slightly AT rich (63% A + T). The sequence AATAAA at nucleotides 2121-2126 apparently is the mRNA polyadenylation sequence since it is followed by a sequence of six A residues at nucleotides 2142-2147.

The accuracy of the DNA sequence coding for tissue factor provided in Formula I was evaluated by comparing the amino acid sequence of the protein predicted from the nucleotide sequence of the open reading frame of the cDNA coding for tissue factor to the amino acid sequence of intact tissue factor, and tryptic digests thereof, purified from human brain and placenta and sequenced by protein sequencing techniques as provided in Example 1.

The portions of the amino acid sequence of tissue factor apoprotein predicted from the sequence of the open reading frame of the cDNA coding for tissue which were confirmed by amino acid sequence analysis as described above are underlined in Formula I. A total of 71.5% of the coding region for the mature protein was confirmed by amino acid sequence analysis and all peptide sequences were matched to predicted peptides. With the exception of residue 208 which was predicted to be glutamic acid from the DNA sequence of the cDNA inserts of both λ10,3 and λ3,4 and glycine based on gas phase sequencing of the peptide encompassing residues 202-215, all of the remaining 262 amino acid residues determined by protein or peptide sequencing agreed with the protein sequence provided in Formula I which was predicted from the sequence of the cDNA coding for tissue factor. The complete amino acid sequence of human tissue factor was not known previously. Thus, the 2147 bp cDNA fragment coding for human tissue factor provides for the synthesis of substantially pure human tissue factor having an amino acid sequence as provided in Formula I.

## Example 4

### Further Characterization the of Human Tissue Factor Apoprotein

The amino-terminal sequence of human tissue factor was determined as described in Example 1 for the apoprotein purified from both brain and placenta. The two preparations gave identical sequences to each other and to the amino acid sequence predicted from the cDNA sequence of the 2147 bp cDNA fragment. Amino acid residues 1-22 of brain tissue factor and residues 1-38 of the placental protein were identical to the predicted sequence of tissue factor provided in Formula I. Each cycle of amino acid sequencing of the intact proteins yielded two PTH amino acids giving overlapping sequences that were out of phase by two residues. This interpretation is consistent with the primary structure as derived from the cDNA sequence (Formula I). Thus, approximately half of the tissue factor apoproteins isolated from brain and placental tissue lacked the first two amino acids. No PTH amino acid was found at position 11, which is believed to be due to glycosylation of the Asn residue at this consensus N-linked glycosylation site (Asn-X-Ser/Thr). (See e.g., Marshall, Biochem. Soc. Symp. 40:17-26, 1974).

The entire amino acid sequence for mature tissue factor shown in Formula I provides for a molecular weight, $M_r$, of the substantially pure tissue factor apoprotein of about 29,600, which is smaller than the $M_r$ of 44-46,000 previously estimated for human tissue factor by SDS-PAGE by Broze et al., J. Biol. Chem. 260:10917-10920, 1985; Guha et al., Proc. Natl. Acad. Sci. 83:299-302, 1986. To examine this discrepancy, tissue factor purified from brain and/or placenta was enzymatically and chemically deglycosylated and the $M_r$ of the resulting apoprotein was determined.

Placental tissue factor (5μg) was chemically deglycosylated by the method of Edge et al., Anal. Biochem. 118:131-137, 1981, using. trifluoromethanesulfonic acid (TFMS). The protein was precipitated with 5 volumes of acetone and pelleted at 5000xg for 30 minutes, after which it was dried in vacuo; 20μl of anisole and 40μl TFMS were then added. The tube was flushed with $N_2$ for 30 seconds, sealed and incubated for 3 hours on ice. The sample was then extracted twice with 3ml ether:hexane (9:1), with 5μl pyridine added to facilitate protein precipitation. Finally, the resulting pellet was extracted once with acetone.

Asparagine-linked carbohydrate was enzymatically cleaved from tissue factor by digestion with endoglycosidase F as described by Steub et al., Biochemistry 24:3587-3592, 1985. Human placental tissue factor (5μg) was precipitated with acetone as described above. The protein pellet was then dissolved in 40μl of a buffer containing 0.5% β-octyl-D-glucopyranoside, 20mM EDTA, and 50mM sodium acetate at pH 6.1, and endoglycosidase F (0.1 unit, Boehringer Mannheim, grade II) was added. After incubation for 16 hours at 37°C, the reaction was terminated by acetone precipitation. The products of chemical and enzymatic deglycosylation were then analyzed by PAGE on the SDS-urea gel system of Swank and Munkres, Anal. Biochem. 39:462-477, 1971.

Untreated tissue factor migrated with an apparent $M_r$ of 42,000 in the SDS/urea PAGE system of Swank and Munkres. This value differed significantly from the apparent $M_r$ of 46,000 observed in the Laemmli SDS-PAGE system and suggested anomalous detergent binding. The TFMS deglycosylated protein gave an apparent $M_r$ of 34,500 in the Swank and Munkres system while the value of the enzymatically deglycosylated material was 33,500. Thus, carbohydrate contributes about 7,500-8,500 daltons to the apparent $M_r$ of tissue factor. It is believed that all the carbohydrate is N-linked (to Asn) since chemical and enzymatic digestions gave essentially the same result.

The complete sequence of human tissue factor provided in Formula I has also been examined for homology with 4668 sequences in the protein sequence database of the National Biomedical Research Foundation, Washington, D.C. The homology search, which was performed using the FASTP program of Lipman et al., Science 227:1435-1441, 1985, revealed no significant amino acid sequence homology between human tissue factor and any protein in the database. In particular, no similarities in primary amino acid sequence were observed between the sequence of tissue factor provided in Formula I and the known amino acid sequences of other clotting proteins, such as thrombomodulin, factor VII, and factor V.

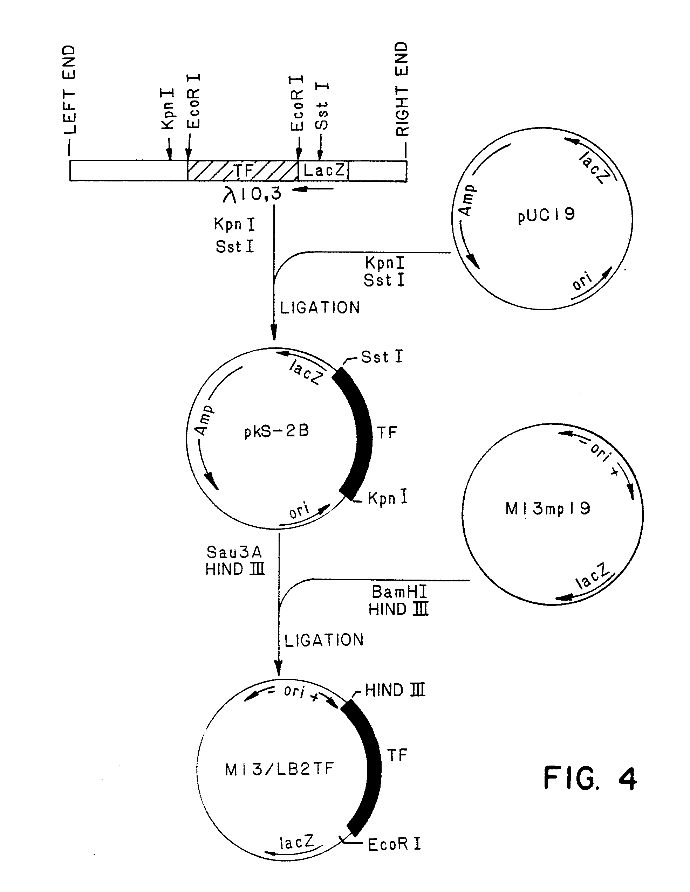

<u>Example 5</u> (Fig. 4)

<u>Construction of Plasmid pKS-2B</u>

Phage λ10,3 DNA was digested with the restriction enzymes Kpn1 and SstI which yielded a DNA fragment comprising the entire 2147 bp cDNA insert coding for tissue factor plus approximately 1000 bp of λ phage DNA from each side of the insert. The approximately 4.15 kb Kpn1/SstI fragment so obtained was then cloned into the lacZ gene of plasmid pUC19 (Norrander et al., Gene 26: 101, 1983; Yanisch-Perron et al., Gene 33:103, 1985) which had been digested with Kpn1 and Sst1. The resulting recombinant plasmid pKS-2B containing the tissue factor gene was then used to transform E. coli 71-18, a host for pUC19. E. coli 71-18/pKS-2B transformants have provided an excellent source of the tissue factor gene for further study and for subsequent construction of expression vectors for production of mature human tissue factor apoprotein (Example 7) and soluble human tissue factor (Example 8) and for localization and characterization of the genomic human tissue factor gene (Example 6).

Example 6

Localization of Human Tissue Factor Coding Sequences in Genomic DNA

Tissue factor coding sequences were localized to an approximately 9.5 kb fragment of genomic DNA by Southern blotting hybridization techniques. This procedure which involves transfer of DNA restriction fragments from an agarose gel after electrophoresis to an appropriate membrane, followed by hybridization with a specific probe, has been used in numerous studies to analyze genome organization and function.

A 1.25 kb Sau3A/HindIII DNA fragment comprising nucleotides 90 to 1340 of the 2147 bp tissue factor cDNA was obtained from plasmid pKS-2B. This 1.25 kb fragment which contained the entire human tissue factor open reading frame (ORF) plus about 110 bp of 5'-untranslated and 343 bp of 3'-untranslated sequences was used to probe for tissue factor coding sequences in total human genomic DNA.

Total human placental genomic DNA was digested with a panel of restriction enzymes. Three (3) µg of each digest were electrophoresed on a 10% agarose gel and then treated with 0.25 M HCl for 15 min. This brief acid treatment following electrophoresis partially depurinated the DNA, breaking it down into smaller pieces that were more readily transferable to the membrane. The acid treatment step was especially important for quantitative transfer of DNA fragments which were more than 5kb in length.

Following acid treatment, the gel was briefly rinsed in distilled water and overlayed with a nylon transfer membrane (Zeta Probe®, Bio Rad). Transfer of the restriction fragments to the membrane was performed by incubation overnight in the presence of a solution of 0.4N NaOH. The transferred DNA became covalently bound to the membrane.

The 1.25 kb Sau3A/HindIII fragment from pKS-2B was used to probe the digested genomic DNA for tissue factor coding sequences. Prior to hybridization the fragment was radiolabelled to a very high specific activity of about $5 \times 10^8$ cpm/µg using the random priming methods described by Feinberg and Vogelstein, Anal. Biochem. 132:6, 1983; Anal. Biochem. 137:266, 1984.

Southern blotting (hybridization) of the labelled 1.25 kb fragment to the genomic restriction fragments bound to the membrane was carried out according to the technique described in Bio Rad Bulletin 1234. Prior to hybridization the membrane was treated at 47°C with 50% formamide; 4xSSPE (20xSSPE = 3.6M NaCl, 0.2M sodium phosphate, pH 7.0, 0.2M EDTA); 1% SDS; 0.5% Blotto; and 0.5mg/ml carrier DNA (salmon sperm). 10% Blotto was prepared by suspending 10g of nonfat powdered milk (Diploma) in 100 ml sterile deionized water and adding sodium azide to a final concentration of 0.2%. The carrier DNA in the prehybridization step was used to minimize non-specific hybridization.

Hybridization was carried out using a hybridization cocktail containing the radiolabelled 1.25 kb tissue factor DNA probe in 47% formamide; 10% dextran sulfate; 3xSSPE; 1% SDS and 0.5% Blotto. Immediately prior to hybridization, the probe was fragmented and denatured by dissolving the radiolabelled 1.25 kb fragment in 0.2M NaOH. Excess carrier DNA was added and the mixture was agitated and centrifuged briefly. The mixture was heated for about 5 minutes at 100°C prior to addition to the hybridization cocktail.

The membrane containing the digested human genomic DNA was incubated with the probe/hybridization cocktail overnight at 47°C ($T_m$-11°C). The blot was then washed at room temperature for 15 min. with 2xSSC/0.1% SDS followed by washing for 15 min. with 0.5xSSC/0.1% SDS. The blot was further washed at 50°C ($T_m$-5°C) for 15 min. with 0.1 SSC/0.1% SDS. Following washing, the blot was exposed overnight at -70°C to an X-ray film (XAR X-Omat, Kodak) with one intensifying screen.

The Southern blot showed the results of hybridization of the 1.25 kb DNA fragment containing the tissue factor coding sequences to various restriction digests of the placental genomic DNA. Lanes 1 and 8 are control digests: phage λ DNA digested with HacIII Ox/HindIII. Lane 2 is BalI digested human DNA; Lane 3 is an EcoRI digest, lane 4 is a HindIII digest; Lane 5 is a Pst1 digest; Lane 6 is a Sau 3A digest; and Lane 7 is a SspI digest. The right side of Fig. 4 shows the electrophorectic mobility of DNA of defined lengths (kb).

It could be seen from the Southern blot that the 1.25 kb tissue factor probe hybridized to two HindIII fragments of 7.0 and 2.546 kb, respectively; to four Pst1 fragments of 4.2, 3.0, 1.5 and 0.7 kb, respectively; and to four SspI fragments of 4.5, 3.8, 0.98 and 0.35, respectively. From this information the size of the chromosomal gene encompassing the DNA coding for tissue factor was determined to be about 9.5 kb. It is also believed that the gene contains at least 3 introns.

<u>Example 7</u>

<u>Expression of Human Tissue Factor</u>

Several host systems have been selected for expression of the structural gene for human tissue factor, e.g. E. coli, CHO cells and insect cells. Expression in these host systems can be accomplished using known techniques to construct expression vectors for expressing tissue factor in the selected host, e.g. M13/pUC vectors for E. coli, mammalian shuttle vectors for CHO cells and baculovirus for insect cells. Expression of tissue factor can also be accomplished in yeast cells using yeast expression vectors, e.g. CYC1, YCpCYC1, YRpCYC1 and dYeCEN3.

<u>(a) Expression of Biologically Active Human Tissue Factor in E. coli</u>

(1) Construction of plasmid pTL8FQ

Plasmid pTL8FQ, which in a suitable host, e.g. E. coli 71-18, expresses the human tissue factor apoprotein, was constructed as follows:

With reference to Fig. 4, the 1.25 kb SauIIIA/HindIII fragment from pKS-2B, obtained as in Example 6, was ligated to BamHI/HindIII digested DNA of the cloning vector M13 mp19 to produce the vector M13/LB2TF.

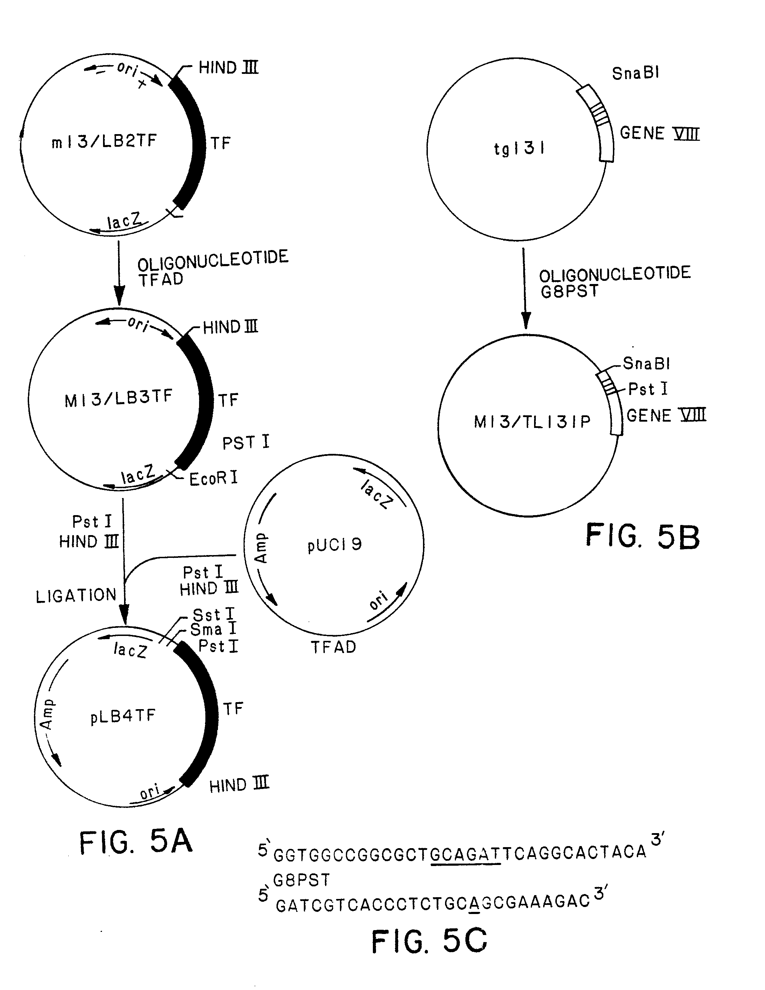

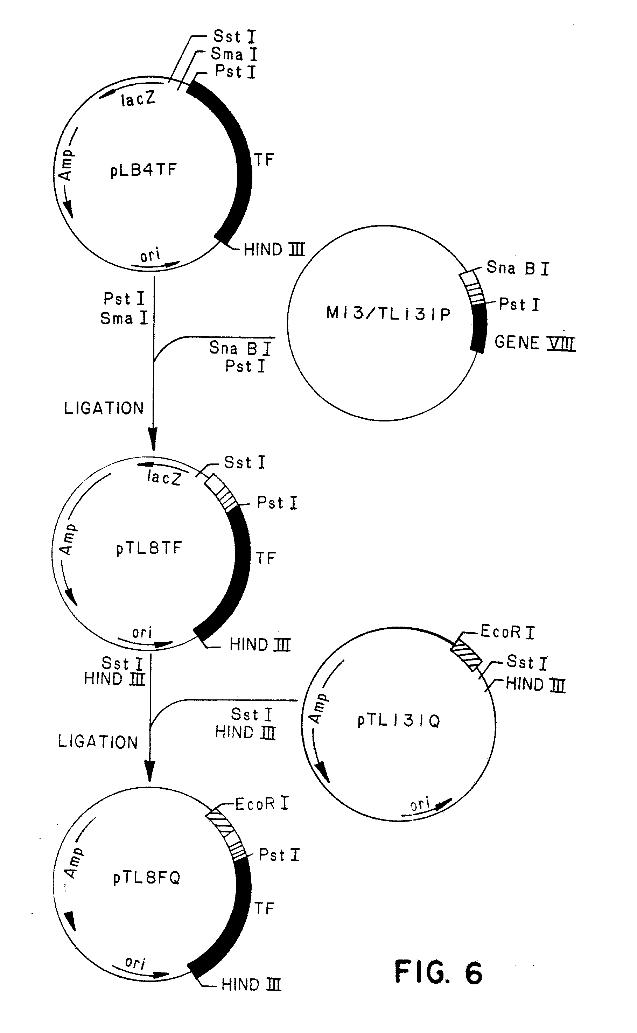

Using the TFAD oligonucleotide sequence shown in Fig. 5c, six bases were then inserted between nucleotides 201 and 202 of the tissue factor coding region in single-stranded DNA from M13/LB2TF by site specific mutagenesis to create a PstI cleavage site in the gene. This latter construct, designated M13/LB3TF (Fig. 6A), was digested with PstI and HindIII and ligated to PstI/HindIII digested pUC19 DNA to produce plasmid pLB4TF, as shown in Fig. 5A.

Next, using the oligonucleotide sequence designated G8PST (Fig. 5C), a PstI site was inserted by site specific mutagenesis into single stranded DNA from bacteriophage tg131 (a derivative of phage M13 which contains multiple cloning sites) just downstream from the M13 gene VIII leader sequence to form M13/TL131P (Fig. 5B). A 100 bp SnaBI/PstI fragment from M13/TL131P was then inserted into the PstI/SmaI site of plasmid pLB4TF to produce plasmid pTL8TF, which contained the DNA coding for the M13 gene VIII leader sequence together with the structural gene for human tissue factor (Fig. 6). Plasmid pTL8TF was then digested with SstI and HindIII to produce a 1252 bp restriction fragment containing the gene VIII leader and tissue factor structural DNA sequences. This fragment was then isolated by agarose gel electrophoresis and cloned into plasmid pTL131Q which had been linearized with SstI and HindIII (Fig. 6).

Plasmid pTL131Q had been constructed by inserting tg131 DNA into the unique PvuII site of plasmid ptac-12, which had been obtained from Dr. John Brosius. Plasmid ptac-12 is a pBR322 derivative and contains the hybrid "tac" (trp/lac) promoter, as disclosed an Amann et al., Gene 25:167-178, 1983. The DNA of phage tg131 was cleaved with BglII and EcoRI converted to blunt-ends using the Klenow fragment of DNA polymerase I from E. coli. Insertion of this DNA into the PvuII site of ptac-12 destroyed the BglII site but restored the EcoRI site in the recombinant pTL131Q. Plasmid pTL131Q now contained the tac promoter, which is inducible with isopropylthiogalactoside (IPTG).

Insertion of the 1252 bp fragment from pTL8TF into pTL131Q resulted in the recombinant expression vector pTL8FQ (Fig. 6), which in a suitable host, e.g. E. coli 71-18, expresses the human tissue factor apoprotein.

### (2) Expression of Human Tissue Factor in E. coli/pTL8FQ Transformants

Plasmid pTL8FQ was then used to transform E. coli 71-18. Because the human tissue factor gene is under the control of the tac promoter in the plasmid, IPTG was used to induce maximum expression of human tissue factor in E. coli 71-18/pTL8FQ transformants. After induction, the cells were sonicated and assayed for tissue factor activity using the two stage clotting assay as in Example 1. Transformant extracts demonstrated a specific activity of tissue factor of about $6 \times 10^4$ units/mg protein which corresponds to about 1ng tissue factor per ml culture. Purified human tissue factor protein as prepared in Example 1 was used as a standard for comparison.

Western blotting analysis with the anti-human tissue factor monoclonal antibody demonstrated the presence of proteins which specifically reacted with the monoclonal anti-tissue factor antibody. Molecular weight standards were run in Lane 1; Lane 2 - total E. coli 71-18/pTL8FQ transformant cell extracts; Lane 3 - Supernatant obtained following centrifugation of sonicated E. coli 71-18/pTL8FQ transformant cells; Lane 4 - Pellet obtained following centrifugation of E. coli 71-18/pTL8FQ transformant cells. The approximate molecular weights of the two bands were 35,000 and 33,000, respectively. The ratio of the 35K to 33K bands was about 5:1. The larger protein is believed to be a protein which contains the M13 gene VIII leader sequence.

### (b) Construction of a Human Tissue Factor Expression Vector For Use in Mammalian Cells

A shuttle vector, pMAM (obtained from CloneTech, cat. #6100-1), capable of transforming E. coli and mammalian cells, such as CHO cells, was selected for construction of a mammalian vector which expresses human tissue factor in a suitable mammalian host cell, e.g. CHO cells. The pMAM vector contains a number of important features which make it suitable for such purposes including the mouse mammary tumor virus (MMTV) promoter, which allows for dexamethasone regulation of transcription and multiple cloning sites adjacent the MMTV promoter, as well as genes for E. coli guanine phosphoribosyl transferase, which codes for a selectable marker to indicate transformation of mammalian cells, and ampicillin-resistance, a selectable marker to detect the plasmid in E. coli transformants. Finally, pMAM also contains a $SV_{40}$ polyadenylation site which allows for synthesis of proteins in mammalian cells.

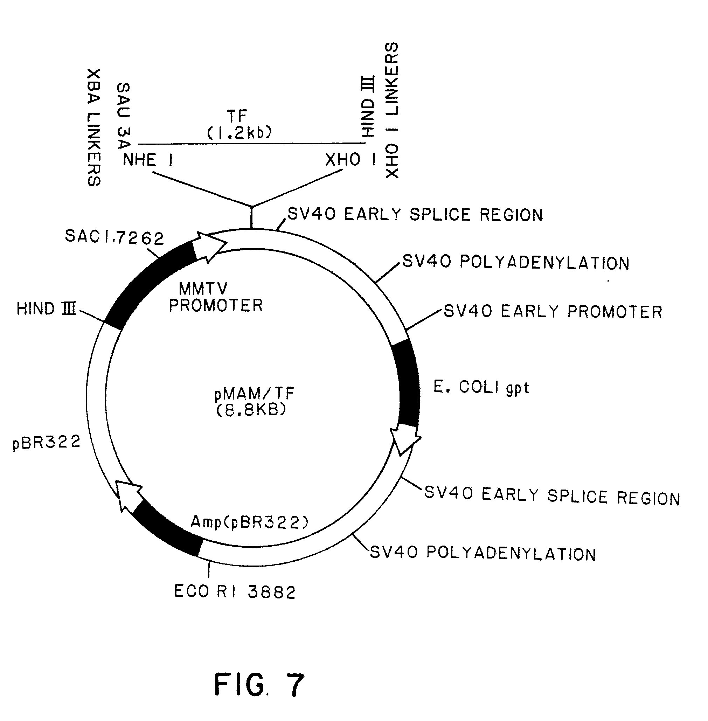

Fig. 7 depicts the pMAM/TF vector which is usable to express human tissue factor in mammalian cells. The vector was constructed by cloning the pKS-2B Sau3A/HindIII fragment described in Example 6 into pMAM as follows: pKS-2B DNA was first digested with HindIII, followed by the addition of XhoI linkers to the HindIII cleavage site. Next, the DNA was digested with SstI, followed by addition of XbaI linkers to the SstI cleavage site. The resulting fragment was cloned into pMAM DNA which had been linearized by cleavage with NheI and XhoI to produce pMAM/TF (Fig. 7).

E. coli XL-1 blue cells (obtained from Stratagene Corp.) are transformed with pMAM/TF. Ampicillin-resistant XL-1/pMAM/TF transformants are screened for the presence of the human tissue factor gene. XL-1 blue transformants which contain the tissue factor coding sequences are selected, the plasmid amplified and obtained for transforming suitable CHO host cells (which lack the gene coding for guanine phosphoribosyl transferase). CHO/pMAM/TF transformants are selected in a medium in which only the cells which have acquired the functional guanine phosphoribosyl transferase can grow, e.g. HAT medium. Transformants growing in the selection medium are then tested for the presence of the human tissue factor DNA sequences and for production of human tissue factor protein.

Example 8

Expression of Soluble Human Tissue Factor Protein

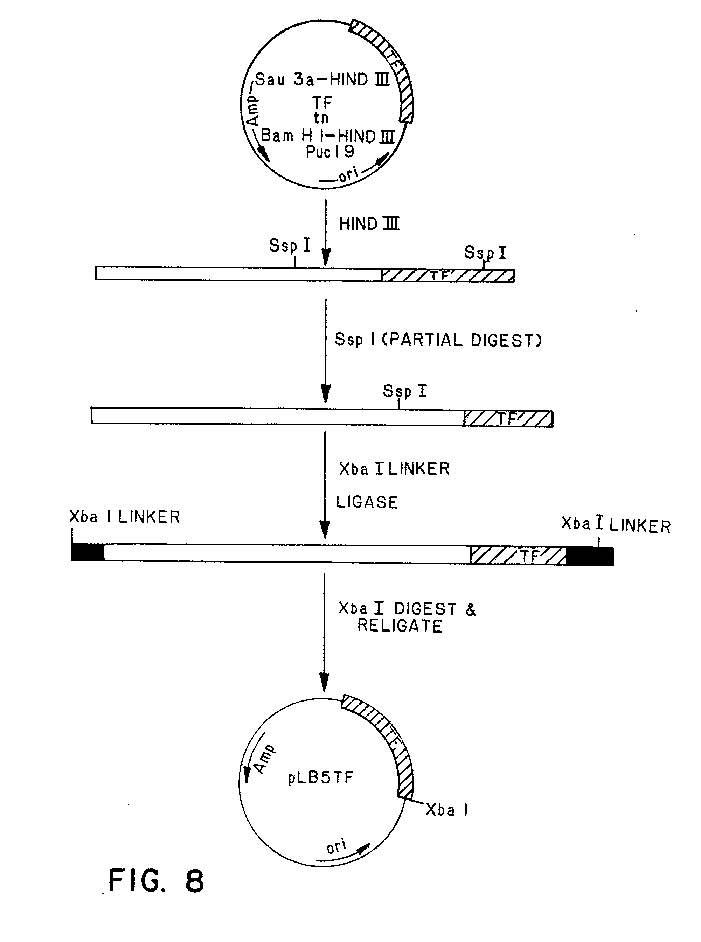

With reference to Fig. 8, the Sau3a/HindIII 1.25 kb DNA fragment containing the entire tissue factor open reading frame (ORF) described in Example 6 was ligated to BamHI/HindIII digested pUC19. The resulting plasmid was then linearized by digestion first with HindIII, followed by a partial digestion with SspI, which produced a cut within the tissue factor ORF at a site approximately corresponding to amino acids 219-220 of the apoprotein sequence (reading from the N-terminal). Following electrophoresis of the digested DNA on an agarose gel, a 3,447 bp fragment was obtained which contains a DNA sequence coding for the extracellular domain (approximately amino acids 1-219/220) of the mature apoprotein. Recessed ends in the fragment were filled in using the Klenow fragment of E. coli DNA polymerase I. Xbal linkers (obtained from New England Biolabs, Cat. #1062), which contained nonsense codons in three reading frames, were ligated to the fragment. The fragment was then digested with Xbal and religated to produce plasmid pLB5TF (Fig. 8).

The Xbal linker adjacent to the 3' side of the truncated tissue factor ORF introduces a stop codon in the plasmid just downstream from the DNA coding for the C-terminal end of the tissue factor extracellular domain. Plasmid pLB5TF thus codes for a truncated soluble human tissue factor which comprises the extracellular domain. In order to amplify the truncated tissue factor coding sequences, plasmid pLB5TF was inserted into and replicated by E. coli 71-18 cells. Plasmid pLB5TF, however, is not an expression vector and E. coli 71-18/pLB5TF transformants do not produce soluble tissue factor.

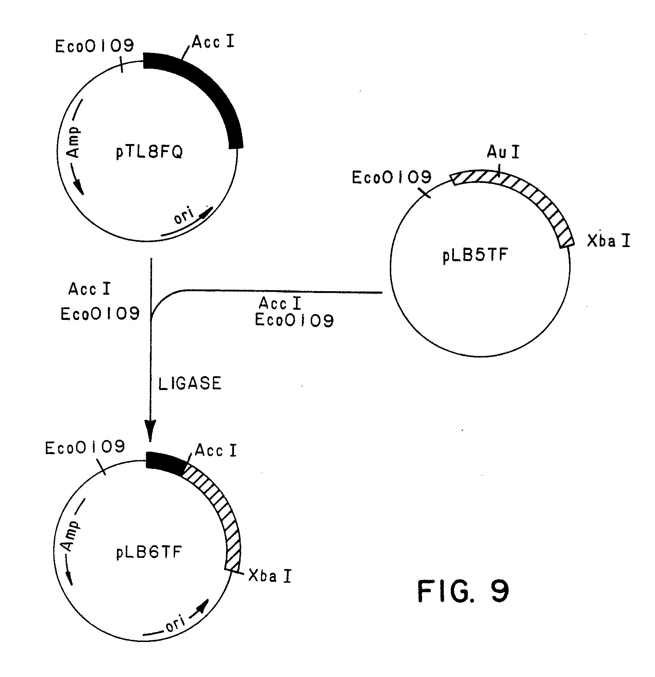

An expression vector pLB6TF which in a transformant host expressed soluble human tissue factor comprising the extracellular domain was produced as follows (see Fig.9):

Plasmid pTL8FQ described in Example 7 was digested with Eco0109 and AccI and electrophoresed. A 500 bp fragment which comprises the DNA sequence coding for the leader sequence of the bacteriophage M13 gene VIII product, the N-terminal amino acids 1-34 of the mature human tissue factor apoprotein (plus an additional ala-asp at the N-terminus) the tac promoter region and a portion of vector DNA was isolated from the agarose gel following electrophoresis and ligated to a DNA fragment from pLB5TF which had been digested with Eco0109 and AccI. The resulting plasmid pLB6TF contained the tac promoter and a DNA sequence coding for the gene VIII leader attached to the DNA sequences coding for the extracellular domain of mature human tissue factor. pLB6TF is an expression vector which, in a suitable host, such as E. coli 71-18, produced soluble active human tissue factor comprising the extracellular domain of the mature human tissue factor apoprotein.

Since the truncated soluble tissue factor expression is under the control of the tac promoter, E. coli 71-18/pLB6TF transformants were induced with IPTG in order to maximize expression of the soluble human tissue factor. The transformant cells were harvested in late log phase and converted to spheroplasts and a supernatant fraction containing periplasmic proteins using standard techniques. Tissue factor activity in the spheroplasts and in the supernatant fraction containing periplasmic proteins (periplasmic fraction) from E. coli 71-18/pLB6TF transformants was assayed using the two stage clotting assay described by Bach et al., J. Biol. Chem. 256: 8324-8331, 1981, as discussed in Example 1.

A low amount of tissue factor procoagulant activity was detectable in both the spheroplast and periplasmic fractions. The activity in spheroplasts was about 100 times higher than the activity in the periplasmic fraction. To increase the activity of the soluble tissue factor in the periplasmic fraction, 100 ml of mixed brain lipid (10 mg/ml) or 100 ml of 0.25% deoxycholate was added to 900 ml of the periplasmic fraction and the mixture was dialyzed overnight against 250 ml of 50 mM Tris-HCL pH 7.5, 100mM NaCl. Aliquots (20 ml) were analyzed for tissue factor activity by the two stage clotting assay. Relipidation of the periplasmic fraction resulted in an increase in tissue factor activity to approximately the level found in the spheroplast fraction.

The soluble tissue factor could be purified from the periplasmic fraction by immunoaffinity chromatography on an immunoadsorbent column prepared with the monoclonal anti-human tissue factor antibody as described in Example 1. Soluble tissue factor was eluted from the column with 0.1M glycine-HCl, pH2.1.

The soluble human tissue factor present in the spheroplasts and in the periplasmic fraction, as well as antibody-purified soluble tissue factor, was also analyzed by Western blot analysis using the monoclonal antibody to human tissue factor. The periplasmic fraction contained a single protein with a molecular weight of about 30,000 which bound the antibody, while two proteins of molecular weights of about 32,500 and 30,000 daltons, respectively, were found in the spheroplasts. The samples were applied to the gel as follows: Lane 1, MW standards; Lane 2, spheroplast fraction; Lane 3, periplasmic fraction; Lanes 4-6, breakthrough fraction from immunoadsorbent column; Lane 8, soluble human tissue factor eluted from immunoadsorbent column with glycine-HCl. The larger protein is believed to be a preprotein containing the M13 gene VIII leader sequence which is subsequently removed from the soluble human tissue factor upon export of the protein into the periplasmic space. The antigenic material appeared to be equally distributed between the spheroplasts and periplasmic fraction. Furthermore, the soluble tissue factor protein is readily isolatable on the immunoadsorbent column.

A second Western blot of the purified product from E. coli 71-18/pLB6TF transformants was run. Lane 1 contained molecular weight standards; Lanes 2 and 3, purified soluble tissue factor. The procoagulant activity of the product was determined to be about $1.9 \times 10^4$ units/mg protein. The soluble tissue factor is thus a much less active procoagulant than the intact apoprotein. The soluble protein, however, does bind to Factor VII and can inhibit the binding of the intact apoprotein to Factor VII.

## Example 9

### (a) Construction of Plasmid pTL8TFA-I

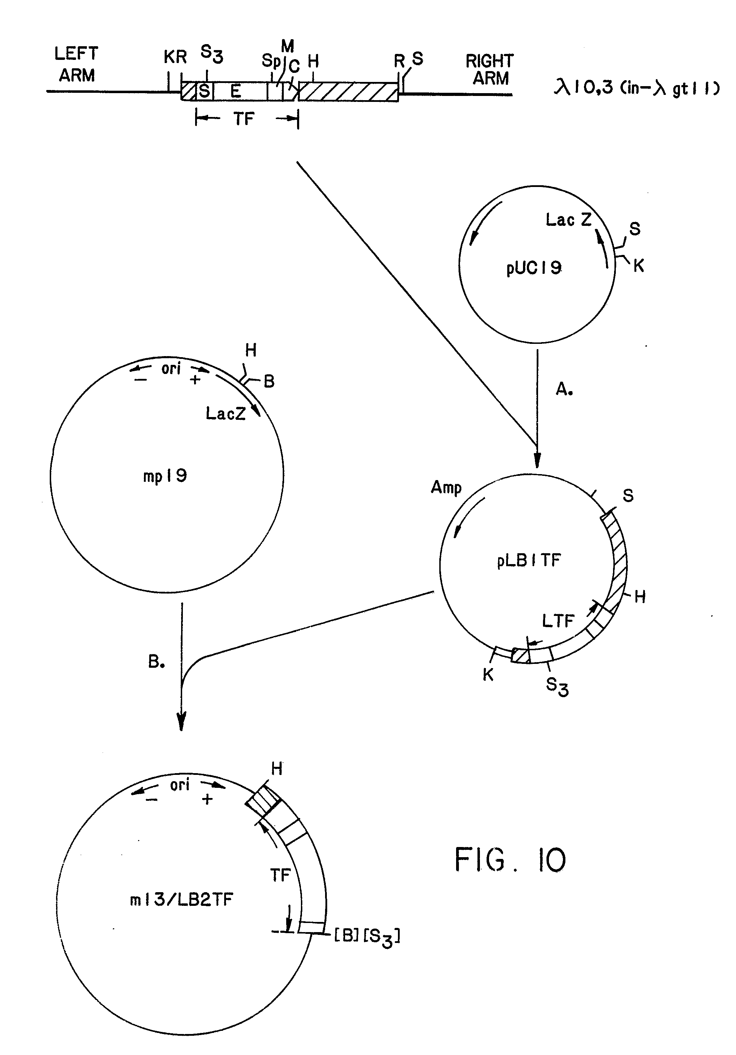

The purified phage DNA (Examples 2-3) was cut with the restriction enzymes Kpnl and Sstl and the 2.2kb fragment was cloned into the plasmid pUC19, Messing, J. (1983) Methods of Enzymol.100 20-78, which had been linearized with Kpnl and Sstl. The resulting plasmid, PLB1TF (Fig. 10), obtained after screening for white cloned colonies, was isolated, purified and restricted with enzymes Sau3a and HindIII to provide a 1252 bp fragment containing the human tissue factor coding region. This fragment, after isolation by gel electrophoresis, was cloned into the RF form of phage mp19 which had been opened at the multiple cloning site by digestion with BamHI and HindIII. The resulting phage, 13/LB2TF, was isolated with the aid of blue/white colony screening (Fig. 5). Oligonucleotide mutagenesis was used to create a pstl site at the 5′ end of the tissue factor structural gene according to the procedure of Kunkel, Kunkel, T.A. (1985) Proc. Nat. Acad. Sci. U.S.A. 82, 488-492. The oligonucleotide used was: 5′ GGTGGCCGGOGCTGCAGATTCAGGCACTACA 3′. The RF form of M13/LB3TF (Fig. 5) was digested with PstI and HindIII and the 1150 bp fragment containing the tissue factor coding sequence was ligated to the linearized form of pUC19 which had been opened with PstI and HindIII to give plasmid pLB4TF (Fig. 5).

To convert the tissue factor structural gene into a form that would allow the tissue factor gene product to be secreted into the periplasmic space, a restriction fragment coding for the bacteriophage M13 gene VIII leader sequence and its corresponding ribosome binding site, was obtained from M13/TL131P by digestion with PstI and Sna BI. This 100 bp fragment was inserted into pLB4TF (Example 7, Figure 5) which had been cut with PstI and Smal using T4 DNA ligase. The resulting plasmid pTL8TF (Example 7, Figure 7) was converted to a vector useful for tissue factor expression by inserting an SstI-HindIII fragment containing the gene VIII leader followed by the tissue factor structural gene downstream from the ptac promoter. This was done by restricting pTL131Q (Example 7, Figure 6) with SstI and HindIII and ligating it with the SstI-HindIII restriction fragment from pTL8TF to five pTL8FQ (Example 7) as shown in Fig. 8. Plasmid pTL8FQ (Example 7) serves as the parent for making various deletions in the tissue structural gene.

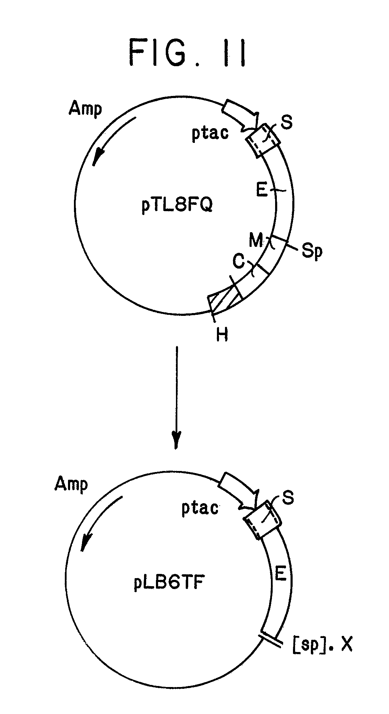

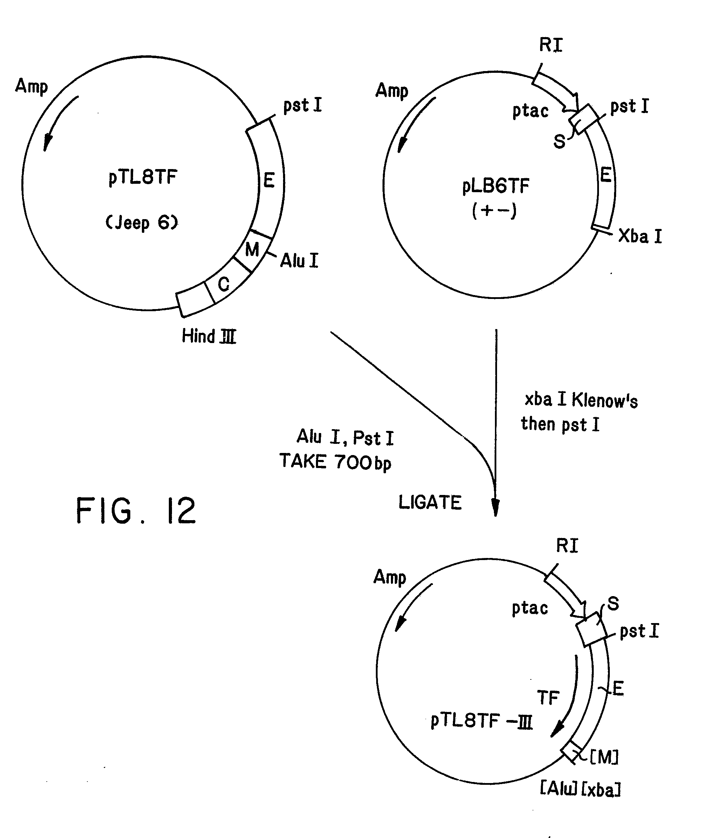

PTL8FQ was digested with HindIII and SspI, and Xbal linker was ligated into the digested pTL8FQ to give pLB6TF, as shown in Fig. 11. pTL8TF was digested with AluI and PstI. The 700 bp fragment containing the coding region for all of the extracellular domain of tissue factor and for six residues of the membrane spanning region was isolated by agarose gel electrophoresis and ligated together with the linearized form of pLB6TF from which the PstI-Xbal fragment had been removed. This was done by first digesting pLB6TF with Xbal, filling in the 5′ overhangs with dNTP's using the E. coli polI Klenow fragment to give blunt ends, then digesting this form with PstI followed by isolation of the large fragment by electrophoresis. The production of ligation of the 700 bp fragment described above with the large fragment of pLB6TF gave pTL8TF-AI, as shown in Fig. 12 with the 37-residue deletion from the COOH terminal of human tissue factor that is referred to as truncated human tissue factor.

### (b) Expression of Truncated Human Tissue Factor

pTL8TF-AI was transformed into E. coli strain 71-18 and allowed to grow in Luria broth at 37 degrees until the cells reached mid-log phase ($D_{570} = 0,5$). Induction of transcription from the ptac promoter was performed by adding isopropylthiogalactoside (IPTG) (2 microliters per ml of culture) and the cells were maintained 25 degrees for four hours. The cells then were harvested by centrifugation, washed once with 0.1 M tris buffer pH 7.5, and resuspended in this buffer to give a dilute slurry, (5 grams of cells/10 ml buffer). 1 ml of $CHCl_3$ was added and the mixture shaken vigorously for two minutes, centrifuged at 7000 rpm and the aqueous layer decanted and retained for subsequent purification. The procedure was repeated, and the aqueous layers were combined.

A 30 ml extract was dialyzed against 2 liters of 10mM tris-cl pH 7.5 containing 1mM benzamidine-HCl in the cold room for 3 hours. The turbid dialyzed suspension was centrifuged at 15000 rpm for 15 min in Beckman refrigerated centrifuge. The precipitate was rejected and the supernatant (30ml) was used for the purification of the truncated human tissue factor.

The 30 ml supernatant was injected using a superloop on a Mono Q column on FPLC system (Pharmacia) (size 1 x 20 cm). The gradient was 10mM tris-cl pH 7.5: 0.3M NaCl in 10mM Tris-cl pH 7.5. The flow rate was 2ml/min and fractions of 2ml/tube were collected at 35% (about 0.105M NaCl), an isocratic step (14ml) was used.

The truncated human tissue factor was eluted at the end of the isocratic step. The truncated human tissue factor was identified using Dot-blot, Western and SDS page analysis.

The pooled peak containing the truncated human tissue factor (#12-14, about 10 ml) was dialyzed against 500ml of 10mM tris-cl pH 7.5 for 3 hours. The dialyzed sample was loaded to the Mono Q column and chromatographed under conditions which were the same as the first column.

The truncated human tissue factor protein was eluted at 40% (about 0.12M Nacl). The pooled peak (#26-30, about 10ml) was concentrated using Centricon 10, to approximately 0.5ml.

The concentrated truncated human tissue factor was injected on a Superose 12 column (1 x 30cm) at a flow rate of 0.25ml/min in a buffer of 10mM tris-cl pH 7.5 containing 0.5mNacl. Fractions of 0.5ml/tube were collected.

Truncated human tissue factor was eluted in fractions #29-30 (total vol 1ml) in a single symmetrical peak. A minor peak containing a low-mol component eluted at #37.

About 4-6 mg of truncated human tissue factor was obtained with approximately 55-70% yield as estimated from gel analysis.

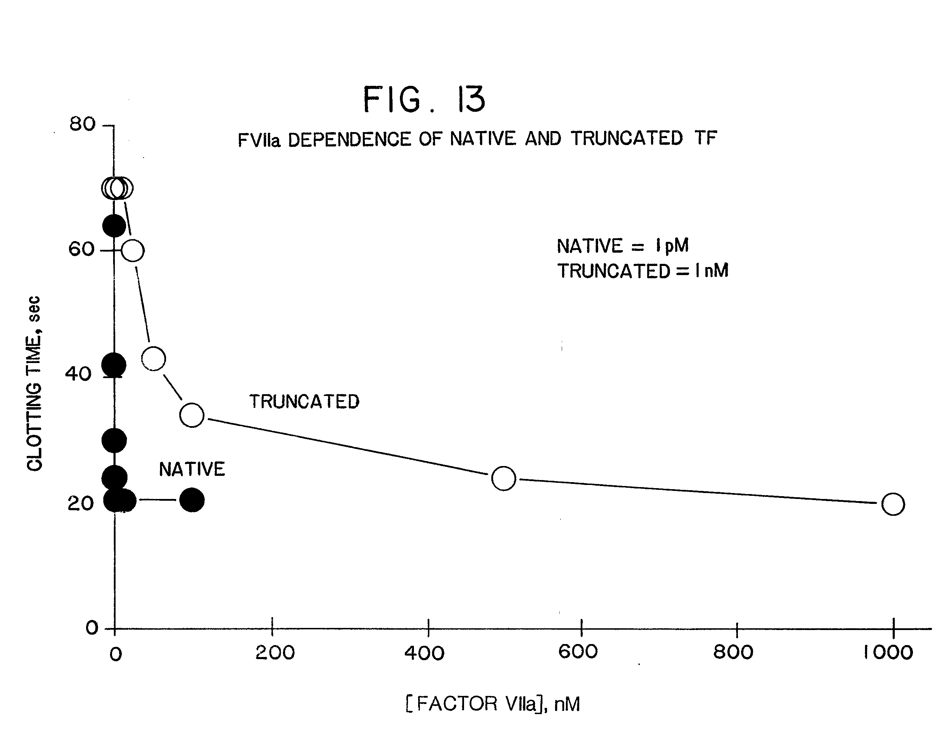

The truncated human tissue factor differs markedly in its response to factor VII as compared to native tissue factor. As shown in Figure 13, native tissue factor gives minimum clotting times with as little as 1 nM factor VII whereas the truncated form shows progressively shorter times even up to 1000 nM factor VII. Thus, truncated human tissue factor will be useful in coagulation tests designed to detect increased (as well as decreased) levels of factor VII activity in plasma.

**Claims**

1. Recombinant cloning vector replicable in host characterized in that it contains within its genome a DNA insert the sequence of which codes for human tissue factor or a portion thereof, preferably which in a suitable host will express said gene coding for human tissue factor or a functional portion thereof.

2. A recombinant cloning vector according to claim 1 characterized in that the vector is selected from the group consisting of plasmids, cosmids, bacteriophage, other vectors replicable in bacteria and vectors replicable in eukaryotes.

3. A recombinant cloning vector according to claim 2 characterized in that the vector is replicable in eukaryotic hosts selected from the group consisting of vaccinia virus, bovine papilloma virus, simian virus$_{40}$, yeast vectors and baculovirus.

4. Recombinant cloning vector according to claim 1 characterized in that the recombinant cloning vector is selected from the group consisting of phage $\lambda$10,3, phage $\lambda$3,4 and plasmid pKS-2B.

5. Recombinant cloning vector according to claim 1 characterized in that the recombinant cloning vector is phage $\lambda$10,3.

6. Recombinant cloning vector according to claim 1 characterized in that the recombinant cloning vector is plasmid pKS-2B.

7. Recombinant vector according to claim 1 characterized in that the vector is pTL8FQ.

8. Recombinant vector according to claim 1 characterized in that the vector is pMAM/TF.

9. Recombinant cloning vector according to any one of claims 1-8 characterized in that the DNA insert codes for soluble human tissue factor or a functional portion thereof.

10. Recombinant cloning vector according to claim 9 characterized in that the DNA insert codes for the portion of soluble human tissue factor which comprises the amino terminal extracellular domain of mature human tissue factor apoprotein.

11. Recombinant cloning vector according to claim 9 characterized in that the DNA insert codes for the portion of soluble human tissue factor which comprises the N-terminal amino acids 1-219/220 of mature human tissue factor, the sequence of which is provided in Formula I.

12. Recombinant cloning vector according to claim 11, in which the recombinant vector is pLB6TF.

13. Recombinant cloning vector according to claim 9 characterized in that the DNA insert codes for the portion of soluble human tissue factor which comprises the N-terminal amino acids 1-227 of mature tissue factor the sequence of which is provided in formula I.

14. Recombinant cloning vector according to claim 13 characterized in that the recombinant vector is pTL8TF-AI.

15. A host organism characterized in that it contains a replicable recombinant cloning vector according to claims 1-14, said vector containing a DNA insert therein the sequence of which codes for human tissue factor or a portion thereof.

16. A host organism according to claim 15 characterized in that the host organism is selected from the group consisting of bacteria, yeast, insect cells, animal cells and human cells.

17. A host organism according to claim 15 characterized in that the organism is E. coli strain K1088.

18. A host organism according to claim 15 characterized in that the organism is E. coli strain 71-18.

19. A host organism according to claim 15 characterized in that the host is CHO cells.

20. A host organism according to claim 15 characterized in that the host is E. coli strain 71-18.

21. A 2147 base pair cDNA fragment coding for human tissue factor, which comprises a nucleotide sequence as provided in Formula I, having an open reading frame extending from an ATG initiation codon at nucleotides 112-114 to a TAA termination codon at nucleotides 997-999, the open reading frame coding for a preprotein of human tissue factor being a single polypeptide chain and having a sequence of 295 amino acids, said preprotein being postranslationally cleaved to substantially pure mature tissue factor which is a single polypeptide chain having an amino acid sequence of 263 amino as provided in Formula I.

22. Substantially pure human tissue factor apoprotein or a functional portion thereof having an amino acid sequence as provided in Formula I.

23. An oligonucleotide probe for identification of DNA coding for human tissue factor, the nucleotide sequence of which corresponds to a sequence of DNA coding for amino acids 24 through 29 of substantially pure human tissue factor having an amino acid sequence as provided in Formula I.

24. An oligonucleotide probe for identification of DNA coding for human tissue factor, the nucleotide sequence of which corresponds to a sequence of DNA coding for amino acids 145 through 159 of substantially pure human tissue factor having an amino acid sequence as provided in Formula I.

25. An oligonucleotide probe for identification of DNA coding for human tissue factor, the nucleotide sequence of which corresponds to a sequence of DNA coding for amino acids 210 through 215 of substantially pure human tissue factor having an amino acid sequence as provided in Formula I.

26. Substantially pure soluble human tissue factor, characterized in that the soluble tissue factor comprises the extracellular domain of mature human tissue factor, the sequence of which is provided in Formula I, or a functional portion thereof.

27. Soluble human tissue factor according to claim 25 in which the soluble tissue factor comprises the N-terminal amino acids 1-219/220 of mature human tissue factor, preferably the N-terminal amino acids 1-227.

NH₂—SGTTNTVAAYÑLTWKSTNFKTILEWEPKPVNQVYTVQISTKSGDWKSKC

EPSNEYLPEGASGTSEVNGAPYSFVRALYTQKVDKVIEDTLDCETDTTYF

100
FTPYLETNLGQPTIQSFEQVGTKVNVTVEDERTLVRRÑNTFLSLRDVFGK

200
RNVTRSPIVAQVSFCYNEGKDVDILFENTNTKATKKGSSSSKWYYLTYILD

EXTRACELLULAR DOMAIN

50

150

MEMBRANE DOMAIN          CYTOPLASMIC DOMAIN
                              250
KSTDSPVECMGQEKGEFRE | IFYIIGAVVFVVIILVIILAISL | HKCRKAGVGQSWKENSPLNVS—COOH

FIG. I

# FIG. 2

EP 0 347 262 A1

OLIGONUCLEOTIDE PROBES

protein sequence (24-29)

Glu-Trp-Glu-Pro-Lys-Pro

coding sequence

5'-GA$^A_G$-TGG-GA$^A_G$-CCX-AA$^A_G$-CCX

Probe #1

3'-CT$^T_C$-ACC-CT$^T_C$-GGX-TT$^T_C$-GG-5'     mixture of 32

---

protein sequence (#145-159)

Asp-Val-Phe-Gly-Lys-Asp-Leu-Ile-Tyr-Thr-Leu-Tyr-Tyr-(Arg/Trp)-Lys

coding sequence

5'-GA$^T_C$-GTX-TT$^T_C$-GGX-AA$^A_G$-GA$^T_C$-$^C_T$TX-AT$^T_C$-TA$^T_C$-ACX-CTX-TA$^T_C$-TA$^T_C$-AG$^A_G$-AA$^A_G$-3'

Probe #2

3'-CTA-CAG-AAA-CCG-TTC-CTG-GAC-TAG-ATG-TGG-GAC-ATG-ATG-GCC-CTT-5'

TF gene

5'-GAT-GTT-TTT-GGC-AAG-GAC-TTA-ATT-TAT-ACA-CTT-TAT-TAT-TGG-AAA-3'
      ★        ★ ★   ★   ★   ★   ★   ★   ★ ★     ★

---

protein sequence (#210-215)

Met-Gly-Gln-Glu-Lys-Gly

coding sequence

5'-ATG-GGX-CA$^A_G$-GA$^A_G$-AA$^A_G$-GGX-3'

Probe #3

3'-TAC-CCX-GT$^T_C$-CT$^T_C$-TT$^T_C$-CC-5'     mixture of 32

FIG. 3

FIG. 4

FIG. 5A

FIG. 5B

5' GGTGGCCGGCGCTGCAGATTCAGGCACTACA 3'
G8PST
5' GATCGTCACCCTCTGCAGCGAAAGAC 3'

FIG. 5C

FIG. 6

FIG. 7

FIG. 8

FIG. 9

FIG. 10

# FIG. II

FIG. 12

# FIG. 13

## FVIIa DEPENDENCE OF NATIVE AND TRUNCATED TF

NATIVE = 1 pM
TRUNCATED = 1 nM

TRUNCATED

NATIVE

CLOTTING TIME, sec

[FACTOR VIIa], nM

EP 0 347 262 A1

| DOCUMENTS CONSIDERED TO BE RELEVANT | | EP 89306176.2 |
|---|---|---|

| Category | Citation of document with indication, where appropriate, of relevant passages | Relevant to claim | CLASSIFICATION OF THE APPLICATION (Int. Cl.4) |
|---|---|---|---|
| D,P, X | <u>WO - A1 - 88/09 817</u><br>(MOUNT SINAI SCHOOL OF MEDICINE OF THE CITY UNI-VERSITY OF NEW YORK)<br>* Claims *<br>-- | 1-27 | C 12 N 15/00<br>C 12 N 1/20<br>C 07 H 21/04<br>C 07 K 13/00<br>//(C 12 N 1/20<br>C 12 R 1:19) |
| P,X | <u>EP - A2 - 0 278 776</u><br>(GENENTECH, INC.)<br>* Claims 1-5,12-19; fig. 2 *<br>-- | 1-3,<br>15,16,<br>21-27 | |
| D,X | PROCEEDINGS OF THE NATIONAL ACADEMY OF SCIENCES OF THE UNITED STATES OF AMERICA, vol. 84, no. 15, August 1987 (Baltimore, USA)<br>E.K.SPICER et al. "Isolation of cDNA clones coding for human tissue factor: Primary structure of the protein and cDNA"<br>pages 5148-5152<br>* Page 5150; fig. 3 *<br>-- | 1,5,<br>11,13,<br>21-27 | |
| P,X | <u>WO - A1 - 88/07 543</u><br>(SCRIPPS CLINIC AND RESEARCH FOUNDATION)<br>* Fig. 2 *<br>-- | 1,11,<br>13,21-<br>27 | TECHNICAL FIELDS SEARCHED (Int. Cl.4)<br><br>C 12 N<br>C 07 H<br>C 07 K |
| D,X | BIOCHEMISTRY, vol. 26, 1987 (Washington DC)<br>E.M.SCARPATI et al. "Human Tissue Factor: cDNA Sequence and Chromosome Localization of the Gene"<br>pages 5234-5238<br>* Abstract; page 5236 *<br>-- | 1,11,<br>13,21-<br>27 | |
| D,X | CELL, vol. 50, no. 1, July 3, 1987 (Cambridge, Mass., USA)<br>J.H.MORRISSEY et al. "Mole- | 1,11,<br>13,21-<br>27 | |

The present search report has been drawn up for all claims

| Place of search<br>VIENNA | Date of completion of the search<br>19-09-1989 | Examiner<br>WOLF |
|---|---|---|

| Category | Citation of document with indication, where appropriate, of relevant passages | Relevant to claim | CLASSIFICATION OF THE APPLICATION (Int. Cl.4) |
|---|---|---|---|
| | cular Cloning of the cDNA for Tissue Factor, the Cellular Receptor for the Initiation of the Coagulation Protease Cascade" pages 129-135 * Page 130; fig. 1 * ---- | | |
| | | | TECHNICAL FIELDS SEARCHED (Int. Cl.4) |

**DOCUMENTS CONSIDERED TO BE RELEVANT**

The present search report has been drawn up for all claims

| Place of search | Date of completion of the search | Examiner |
|---|---|---|
| VIENNA | 19-09-1989 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document. but published on, or after the filing date
D : document cited in the application
L : document cited for other reasons

& : member of the same patent family. corresponding document

EPO Form 1503 03 82